# EUROPEAN PATENT SPECIFICATION

(11) **EP 2 739 659 B1**
(45) Date of publication and mention of the grant of the patent: **17.06.2015**
(21) Application number: 12762693.5
(22) Date of filing: 03.08.2012
(51) Int. Cl.: C08F 220/06, C08F 222/10, C08F 220/10

(54) **IONIC/IONOGENIC COMB COPOLYMER COMPOSITIONS AND PERSONAL CARE PRODUCTS CONTAINING THE SAME**
IONISCHE/IONOGENE KAMMCOPOLYMERZUSAMMENSETZUNGEN UND KÖRPERPFLEGEPRODUKTE DAMIT
COMPOSITIONS À BASE DE COPOLYMÈRES EN PEIGNE IONIQUES/IONOGÈNES ET PRODUITS D'HYGIÈNE PERSONNELLE EN CONTENANT

(30) Priority: 03.08.2011 US 201161514528 P
(43) Date of publication of application: 11.06.2014
(73) Proprietor: Air Products and Chemicals, Inc., Allentown, PA 18195-1501 (US); Landec Corporation, Menlo Park, California 94025 (US)
(72) Inventor: GODDARD, Richard Joseph, Fogelsville, Pennsylvania 18051 (US); NOOR, Mussarat, Pittstown, New Jersey 08867 (US); SCHAFER, Julian Everett, Montara, California 94037 (US); BITLER, Steven Paul, Menlo Park, California 94025 (US); TAFT, David Dakin, Atherton, California 94027 (US); ZHENG, Shiying, Center Valley, Pennsylvania 18034 (US)
(74) Representative: Kador & Partner
(86) International application number: PCT/US2012/049517
(87) International publication number: WO 2013/020049

(56) References cited:
- EP-A1- 0 577 526
- EP-A1- 1 167 505
- EP-A1- 1 607 416
- EP-A2- 0 217 485
- EP-A2- 0 335 624
- EP-A2- 0 979 833
- JP-A- 2000 016 921
- JP-A- 2004 359 905

## Description

### CROSS-REFERENCE TO RELATED APPLICATIONS

This Application claims the benefit of U.S. Patent Application No. 61/514,528, filed on August 03, 2011. The disclosure of Application No. 61/514,528 is hereby incorporated by reference.

### BACKGROUND OF THE INVENTION

The present disclosure relates generally to the field of copolymers and more particularly to ionic/ionogenic comb copolymer compositions and their use in personal care and other products.

Polymeric thickeners are sometimes used for cosmetic and other personal care products. Many known thickeners use hydrophilic polymers with anionic functionality derived either from high levels of acid monomers, such as [meth]acrylic acid or 2-acrylamido-2-methylpropane sulfonic acid (AMPS), or from polyethylene glycol (PEG) content derived either from ethoxylated monomers or grafting. In some cases, thickening efficiency is enhanced with crosslinking and/or hydrophobic modification, often by the inclusion of associative monomers that add a limited amount of hydrophobic character. While these polymers offer compatibility with anionic surfactants used for shampoo and other cleansing agents, they offer limited deposition and wash-off resistance.

Synthetic anionic polymers often include cross-linking, and/or are high molecular weight polymers with only limited deposition and sensory benefit. Overall, the use of such prior modifiers has generally been unsatisfactory as they are designed for the primary benefit of thickening and fail to provide more than a modest benefit across other categories of desirable attributes, resulting in the need to incorporate multiple different modifiers and other additives to overcome their respective individual deficiencies.

Anionic polymer thickeners particularly suitable for use in applications outside of personal care have been described at U.S. 3,652,497, U.S. 4,138,381, EP0003235, EP0013836, U.S. 4,514,552 and U.S. 4,792,343. Other anionic polymerthickeners for use in aqueous systems containing inorganic salts are described in U.S. 3,915,921. And related anionic polymer thickeners for use in personal care applications are disclosed in U.S. 2001049419, U.S. 2003207988 and WO09090204.

The disclosure of the previously identified patents and patent applications is hereby incorporated by reference.

However, it is believed that none of these references disclose or recognize a composition that achieves both sensory and deposition benefits, combined with substantial rheology enhancement in many cosmetic formulations, nor the ability to depositor retain active ingredients. Existing anionic polymers do not significantly improve deposition/retention of actives. Furthermore, in most of the references that employ associative monomers in combination with ionic or ionogenic monomers, the ratio of ionic or ionogenic groups to associative groups is very high.

It would be advantageous to provide an ionic/ionogenic copolymer that could achieve multiple benefits and provide a balance of desirable properties in personal care and other products, and particularly to accomplish both increased deposition effectiveness and good sensory attributes.

Exemplary embodiments of the present invention overcome these and other drawbacks by providing ionic/ionogenic copolymer compositions that provide a balance of desirable characteristics for use in personal care products to achieve superior results, while decreasing the number of separate ingredients that must be employed to address each desired attribute through the recognition of a relationship between the weight of lipophilic associative groups and concentration of ionic/ionogenicgroups in the backbone of the copolymer.

### BRIEF SUMMARY OF THE INVENTION

In one embodiment, an ionic or ionogenic comb copolymer comprises
A) one or more repeating units derived from olefinically unsaturated anionic or anionogenic orzwitterionic comonomers; and
B) one or more repeating units having the formula 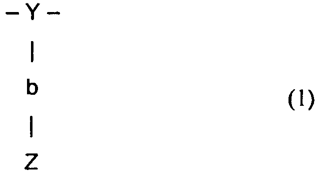
in which Y is a moiety forming part of the copolymer backbone, Z is a moiety that exhibits association in the presence of other Z moieties or with other moieties within the ultimate formulation in which the copolymer will be employed, and b is a bond or moiety that links the Z moiety to the Y moiety, and wherein a single olefinically unsaturated comonomer may impart both the required anionic or anionogenic or zwitterionic groups of comonomer A and repeating units of formula (1).

The copolymer generally further includes one or more repeating units derived from at least one of the following categories:
C) acrylamide monomers;
D) one or more olefinically unsaturated hydrophilic monomers that are not A, B or C; or
E) one or more olefinically unsaturated monomers that are not A, B, C or D.

In another embodiment, the ionic/ionogeniccomb copolymer has a concentration of ionic and ionogenic monomers incorporated into the copolymer of between about 10 and about 450 milliequivalents (meq) per 100 grams of polymer, the weight of associative groups Z in the copolymer is between about 3 and about 40 grams per 100 grams of polymer, and the ratio of associative groups Z to ionic and ionogenic monomers is between about 0.025 and about 1.3 g/meq.

In yet another embodiment, the ionic/ionogenic comb copolymer has a concentration of ionic and ionogenic monomers incorporated into the copolymer of between about 10 and about 450 meq per 100 grams of polymer, the weight of associative groups Z in the copolymer is between about 3 and about 40 grams per 100 grams of polymer, and the viscosity of a 3% by weight (solids) solution of the copolymer in distilled water at pH 7.0 ± 1.0 is less than about 250 centipoise (cP). Throughout the present disclosure, pH is determined at room temperature of 20 to 24 °C with a Beckman 200 Series Meter and Thermo Scientific pH Combination Electrode calibrated against pH 4, pH 7 and pH 10 buffer solutions.

The ionic/ionogeniccomb copolymers disclosed herein may be incorporated into any cosmetically acceptable media and in some embodiments a personal care product comprises a cosmetically acceptable base media and between about 0.1 to about 20% by weight of the ionic/ionogenic comb copolymer.

In certain embodiments, the personal care product further comprises an active ingredient, wherein the ionic/ionogenic comb copolymer increases the deposition of the active ingredient to a keratinous substrate by at least about 10% over a personal care product having the same formulation except for the ionic/ionogeniccomb copolymer. In still other embodiments, the use of the ionic/ionogenic comb copolymer can itself demonstrate deposition to keratinous substrates.

In certain embodiments, the personal care product comprises a comb polymer wherein the polymer is not crosslinked (as defined below) and has relatively low water solubility (as defined below).

Compositions and methods in accordance with exemplary embodiments can be used in personal care applications to provide multiple benefits of rheology modification/thickening (e.g., Example 32, Example 50, Example 90, Example 99, Example 277, Example 311, and Example 336, among others), deposition and retention of actives (e.g., Examples 21-40 and Examples 81-100), water resistance, and sensory (conditioning, slip, silky feel) benefits (e.g., Example 47, Example 50, Example 51, Example 52, Example 57, and Example 59, among others), all of which can be achieved using a single polymer in cosmetic media. Certain exemplary embodiments exhibit good stability and provide multiple benefits of rheology modification/thickening, deposition and retention of actives, water resistance, and sensory benefits in low pH cosmetic media (e.g., Example 12 and Example 16). By low pH cosmetic media, it is meant a cosmetic media having a pH of less than about 4.5, and particularly having a pH less than about 4.0.

Exemplary embodiments provide an ionic/ionogeniccomb copolymer having a particular combination of repeating units to provide rheology modification with a fatty or lipophilic component of a personal care product. The combination of a sufficient amount of hydrophilic monomer for compatibility in aqueous formulations with substantial hydrophobic content both enhances thickening by association and dramatically improves deposition and wash-off resistance.

Other features and advantages of the present invention will be apparent from the following more detailed description of exemplary embodiments that illustrate, by way of example, the principles of the invention. The features and embodiments disclosed herein can be used alone or in combination.

### DETAILED DESCRIPTION OF THE INVENTION

### Description of copolymer and its constituent groups

Embodiments are directed to ionic and ionogenic comb copolymers that comprise repeating units containing associative moieties and that are formed from at least two categories of monomers, and typically at least three categories of monomers. In some embodiments, four or five categories may be employed. Embodiments are also directed to personal care products and other useful compositions that include these copolymers as an ingredient

The ionic or ionogenic comb copolymer comprises
A) one or more repeating units derived from olefinically unsaturated anionic or anionogenic orzwitterionic comonomers; and
B) one or more repeating units having the formula 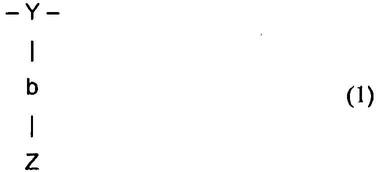
in which Y is a moiety forming part of the copolymer backbone, Z is a moiety that exhibits association in the presence of other Z moieties or with other moieties within the ultimate formulation in which the copolymer will be employed, and b is a bond or moiety that links the Z moiety to the Y moiety. By "exhibits association" is meant that the Z moieties experience weak attractive bonding (e.g. Van derWaals interaction, hydrogen bonding) that cause them to associate with one another or with other moieties within the ultimate formulation, as will readily be appreciated and understood by those of ordinary skill in the art. The association can result in the formation of a dynamic three-dimensional network structure of micelles or having micelle-like features; although the associations in the network are dynamic and the forces are weak, the associations often have sufficient lifetime and strength to exhibit increased viscosity. In some embodiments a single olefinically unsaturated comonomer may impart both the required anionic or anionogenic or zwitterionic groups of comonomer A and repeating units of formula (1).

In typical embodiments, the copolymer further includes one or more repeating units derived from at least one of the following categories:
C) acrylamide monomers;
D) one or more olefinically unsaturated hydrophilic monomers that are not A, B or C; or
E) one or more olefinically unsaturated monomers that are not A, B, C or D.

That is, in typical embodiments, the copolymer includes at least one each of A and B and further includes repeating units derived from at least one of C, D or E. In other embodiments, the copolymer includes at least one each of A and B and further includes repeating units derived from monomers from two of the C, D, and E categories, while in a still other embodiment, the copolymer includes at least one each of A and B along with repeating units derived from monomers from all three of groups C, D and E. It will be appreciated that in each case, multiple members from the same group may also be present.

The ionic and ionogenic monomers from which the group A constituent is selected can be any anionic or anionogenic monomer. Particularly suitable anionic monomers include salts of at least one of monomers with carboxylic or dicarboxylic acid groups, anhydrides, and salts of sulfonic or phosphonic acid, as well as salts of half-esters of dicarboxylic acids. Such monomers include at least one member selected from the group consisting of ammonium [meth]acrylate, sodium itaconate, sodium citraconate, sodium maleate, sodium [meth]acrylate, sodium fumarate, sodium styrene sutfonate, and ammonium or sodium acrylamidomethylpropane sulfonate, all by way of example. Suitable anionogenic monomers include any monomer that can be base-neutralized to form anionic groups, either prior, during or subsequent to copolymer formation. Examples of anionogenic monomers include at least one member selected from the group consisting of [meth]acrylic acid, itaconic acid, crotonic acid, citraconic acid, maleic acid, methyl maleate, butyl maleate, vinyl sulfonic acid, vinyl benzoic acid, styrene sulfonic acid, 3-sulfopropyl methacrylate, vinyl phosphonic acid, allyl phosphonic acid, fumaric acid, mesaconic acid, glutaconic acid, maleic anhydride, citraconic anhydride, and itaconic anhydride. Optionally, the A group can comprise AMPS or acrylamidomethylpropane sulfonic acid.

The pH of the ionic/ionogenic comb copolymers can be adjusted by any suitable organic acid, mineral acid or alkaline materials prior to and/or subsequent to its addition to a personal care or other formulation. When pH adjustment to a more acidic state is desired, various acidic materials may be used including for example one or more of hydrochloric acid, hydrobromic acid, sulfuric acid, phosphoric acid, acetic acid, citric acid, tartaric acid, gluconic acid, and the like. When pH adjustment to a more alkaline state is desired, various alkaline materials may be used including for example one or more of sodium hydroxide, potassium hydroxide, ammonium hydroxide, ethanolamine, diethanolamine, triethanolamine, 2-amino-2-methyl-1-propanol, triethylamine, and the like.

In some embodiments if desired, zwitterionic and/or amphoteric monomers may also be employed as group A monomers. Zwitterionic monomers or repeats are those which contain both a formal positive and negative charge on different atoms; although they carry a net charge of zero, they are considered ionic monomers. Examples of zwitterionic monomers that may be used as group A monomers include those having pendant carboxybetaine, phosphobetaine, or sulfobetaine moieties; specific examples include 2-methacryloxyethyl phosphorylcholine, 2-(methacryloyloxy)ethyl-2'-(trimethylammonium)ethyl phosphate inner salt, N,N-dimethyl(methacryloyloxyethyl)ammonium propanesulfonate, N,N-dimethyl(methacrylamidopropyl)ammonium carboxymethyl inner salt, N,N-dimethyl(methacrylolyethyl)ammonium carboxymethyl inner salt, N,N-dimethyl(methacrylamidopropyl)ammonium propionate,1(4(4'-vinylbenzyloxy)butane)-2'(trimethylammonium)ethyl phosphate salt, and [3-(methacryloylamino)propyl]dimethyl(3-sulfopropyl) ammonium hydroxide salt Amphoteric monomers are ionogenic monomers which contain both acidic and basic groups.

In the repeating units of Group B, the associative moiety Z can be incorporated into the comb copolymer as a component of a monomer used in the copolymerization or it may result from a reaction of the associative group and a functional monomer during or subsequent to polymerization. Thus, it may be possible in some cases for the moiety Y of the copolymer backbone in the repeating unit from which the associative moiety Z extends to be a repeating unit derived from the monomer of groups A, C, D or E, but which falls into group B by virtue of its subsequent linkage to the associative moiety Z.

The Z moiety is generally hydrophobic in nature, and can be alkyl, aryl, aralkyl, fluoroalkyl, silicone orsilane. For example, hydrocarbon Z groups include saturated, unsaturated, linear, branched or cyclic alkyl, aryl or aralkyl groups having in the range of 8 to 50 carbon atoms, in one embodiment having in the range of 10 to 22 carbon atoms, in another embodiment having in the range of 10 to 18 carbon atoms, and in yet another embodiment, the groups have in the range of 12 to 18 carbon atoms. For example, Z alkyl components include but are not limited to at least one member selected from the group consisting of alkyl groups (such as octyl, nonyl, decyl, undecyl, dodecyl, tridecyl, tetradecyl, pentadecyl, hexadecyl, heptadecyl, octadecyl, nonadecyl, eicosyl, arachinyl, and behenyl) derived from fatty alcohols, fatty amines, or fatty acids. Suitable Z alkyl components also include mixtures of such compounds, and where mixtures of alkyl components are used the ranges of carbon atoms indicated above are based on the weight average of the mixture. Exemplary commercial mixtures include alcohols sold under the tradenames Alfol® and Novel®from Sasol, alcohols sold under the tradename Neodol®from Shell, as well as alcohol mixtures sold by Baker Hughes under the tradename Unilin®alcohols, in which the average chain length is C25, C30, C40 or C50, as well as alcohols derived from coconut or other natural oils. Also included may be predominantly linear alkenyl chains including at least one member selected from the group consisting of dodecenyl, tridecenyl, tetradecenyl, pentadecenyl, hexadecenyl, octadecenyl, and docosenyl. In some cases it is desirable to minimize or eliminate associative groups with aromatic rings and, in one aspect of the invention, Z is substantially free of such groups. By "substantially free" it is meant that Z comprises or consists essentially of at least one member selected from the group consisting of alkyl, fluoroalkyl, silicone and silane.

Silicone containing Z groups can be linear, branched or cyclic having between 3 and 25 linked SiO units. In one embodiment, it has between 3 and 8 SiO units, and in another embodiment has between 3 and 5 SiO units. Fluoroalkyl containing Z groups include fluorocarbon groups having in the range of 6 to 50 carbons, and in the range of 8 to 30 carbons in another embodiment.

Examples of the linkage b include covalent bonds formed by at least one of ester, carbonyl, amide, amine oxide, hydrocarbon, amino, ether, and polyoxyalkylene groups linking the copolymer backbone to the associative group Z. The linkage b may also include urethane and urea functions as described for example in U.S. 4,514,552, U.S. 4,600,761, U.S. 5,015,711 and WO09090204; the disclosure of which is hereby incorporated by reference. The linkage b may also occur through ionic salt linkages.

The repeating units of Group B may be further characterized as surfactant repeating units or hydrophobic repeating units. Surfactant repeating units are units of formula (1) that can be cleaved, experimentally or theoretically, at the Y-b chemical bond, or a chemical bond within the b linkage, to yield a surfactant that includes the Z moiety. The structure, characteristics and properties of surfactants are well known throughout the literature, and example surfactants include compounds that reduce the surface tension of the water-air interface by 20 dynes/cm at a concentration less than or equal to 1 x 10⁻³ M and are capable of forming micelles. Hydrophobic repeating units are units of formula (1) that are not surfactant repeating units. The ionic/ionogenic comb copolymer can be comprised of repeating units of Group B that include only surfactant repeating units, that include only hydrophobic repeating units, or that include both surfactant repeating units and hydrophobic repeating units. In certain embodiments, the repeating units of Group B are surfactant repeating units only.

The following is a generic structure of a monomer that forms a repeating unit of Group B, having an optional polyoxyalkylene component:

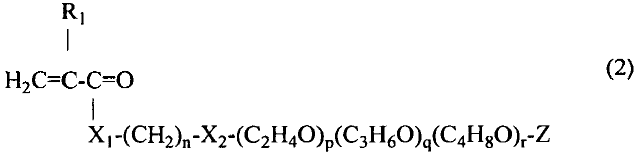

X₁ and X₂ are independently either O, S, NH, or NHCOO; R₁ is either H or CH₃; n is between 0-6 (in which X₂ is omitted when n=0); p is between 0-50, q is between 0-50 and r is between 0-50. Repeating units of p, q and r may be incorporated in random, alternating, block or gradient structures. Z is any moiety as previously described. In one embodiment, wherein p = q = r = 0 the weight percentage of the Group B monomer can be less than about 50wt.%, in some cases less than about 30wt.%, in another case, less than about 20wt% and in a further case, less than about 5wt.%. For example, in one aspect of this embodiment, at least one of p, q or r is between one and fifty (e.g., greater than or equal to 1). For example, in another aspect of this embodiment, p is between 1 and 50, in some cases between 3 and 30 and in other cases between 2 and 15. In certain embodiments, X₁=O, n=q=r=0, p is between 3 and 30 and Z is an alkyl group having in the range of 10 to 18 carbon atoms. In another embodiment, R₁=H, X₁=O, n=q=r=0, p is between 2 and 10 and Z is an alkyl group having in the range of 10 to 16 carbon atoms. Examples of monomers of structure (2) that will form Group B repeating units include at least one member selected from the group consisting of stearyl poly(oxyethyl)₂₅ methacrylate, lauryl poly(oxyethyl)₄ acrylate, octyl poly(oxypropyl)₆poly(oxyethyl)₁₂-N-methyl acrylamide, N-octadecylmethacrylamide, and lauryl [meth]acrylate, for example.

The following is a generic structure of a monomer that forms a repeating unit of Group B having polyalkylamine groups:

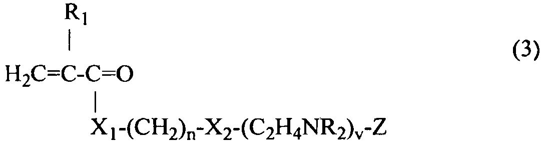

X₁ and X₂ are independently either O, S, NH, or NHCOO; R₁ and R₂ are independently either H or CH₃; n is between 0-6 (and X₂ is omitted when n=0); and v is between 1-50. The Z moiety is as previously described.

Monomers that impart both the required anionic oranionogenic or zwitterionic groups of comonomer A and repeating units of formula (1) include for example monomers formed from the reaction of a dicarboxylic acid or anhydride and a Z group that is reactive with carboxylic acid or anhydride, examples of which include Z groups with hydroxyl or amino functionality. Examples of monomers that include repeating units of comonomer A and repeating units of formula (1) are described in U.S. 3,657,175 and U.S. 4,616,074; the disclosure of which is hereby incorporated by reference. The following generic structures (4A) and (4B) are monomers that include repeating units of comonomer A and repeating units of formula (1):

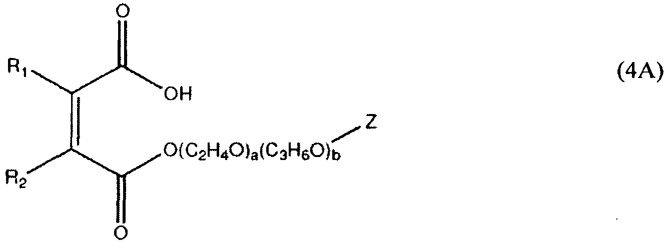

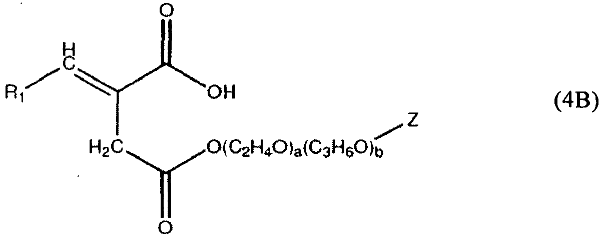

R₁ and R₂ are independently either H or CH₃; a is between 0-50 and b is between 0-50. Repeating units of a and b may be incorporated in random, alternating, block or gradient structures. Z is any moiety as previously described. In one embodiment of structure (4A), R₁ is H or CH₃, R₂ is H, b=0, Z is an alkyl group having in the range of 8 and 18 carbon atoms, and a is between 0-30. In another embodiment a is between 2-20, and in yet another embodiment a is between 4-12.

The acrylamide monomers of Group C may be any monomer having the following formula

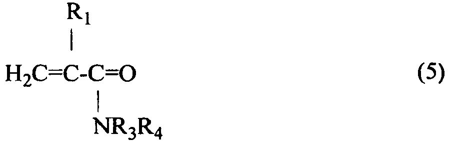

R₁ is either H or CH₃, R₃ and R₄ are independently H, C1-C6 alkyl, C1-C6 alkenyl, C1-C6 alkoxyalkyl, or alkylaminoalkyl. Exemplary Group C monomers include, but are not limited to at least one member selected from the group consisting of acrylamide, methacrylamide, N-methylacrylamide, N-methylmethacrylamide, N,N-dimethylacrylamide, N,N-dimethylmethacrylamide, N,N-diethylacrylamide, diacetoneacrylamide, N,N-dimethylaminopropylacrylamide, N-butoxymethylacrylamide, N-ethoxymethylacrylamide, N-n-butylacrylamide, N-tertbutylacrylamide, N-isopropylacrylamide, N-methylolacrylamide, N-methoxymethylacrylamide, N-ethylacrylamide, N-(3-methoxypropyl)acrylamide, N-n-propylacrylamide, N-trimethylbutylacrylamide, N-isooctylacrylamide, N-acetylmethacrylamide, N-butoxymethylmethacrylamide, N,N-dibutylaminopropylmethacrylamide, N,N-dimethylaminopropylmethacrylamide, N,N-diethylmethacrylamide, N-(2,2-dimethoxyethyl)methacrylamide, N,N-dimethylaminoethylmethacrylamide, N-ethylmethacrylamide, N-methoxymethylmethacrylamide, N-(3-methoxypropyl)methacrylamide, and N-methylolmethacrylamide.

Monomers of Group D are any olefinically unsaturated hydrophilic monomers that do not fall within Group A, B or C, and generally have a water solubility at 25 °C greater than about 50 g/L.

Group D monomers may also include nonionic monomers with the following structure, having an optional polyethyleneglycol (PEG) component:

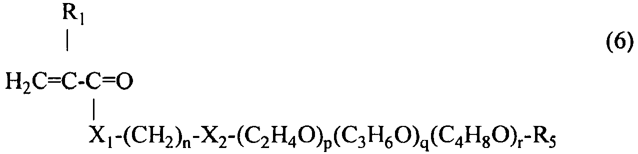

X₁ and X₂ are independently either O, S or NH; R₁ is either H or CH₃; R₅ is either H or linear or branched alkyl with 1 to 4 carbon atoms; n is between 0-6 (and X₂ is omitted when n=0); p is between 0-50, q is between 0-50, and r is between 0-50. Repeating units of p, q and r may be incorporated in random, alternating, block or gradient structures.

Examples of Group D monomers having the structure of (6) include at least one member selected from the group consisting of hydroxyethyl [meth]acrylate, hydroxypropyl [meth]acrylate, hydroxybutyl [meth]acrylate, hydroxyhexyl [meth]acrylate, and corresponding [meth]acrylamides including hydroxyethyl [meth]acrylamide, hydroxypropyl [meth]acrylamide, hydroxybutyl [meth]acrylamide, hydroxyhexyl [meth]acrylamide, poly(oxyethyl)₁₀ methacrylate, and methyl poly(oxyethyl)₈ acrylate.

Other exemplary nonionic monomers within group D include at least one of glycerol monomethacrylate, trimethylolpropane mono[meth]acrylate, pentaerythritol mono[meth]acrylate, methoxyethyl [meth]acrylate, vinyl alcohol (from vinyl acetate), vinylpyrolidinone, and N-vinylformamide.

Monomers of Group E are broadly any other olefinically unsaturated monomers that do not fall into groups A through D. Exemplary monomers primarily include at least one member selected from the group consisting of mono-unsaturated monomers such as C1-C4 alkyl [meth]acrylates, C1-C4 alkyl fluoro [meth]acrylates, vinyl esters, vinyl caprolactam, alkylvinyl ethers, vinyl amides, styrene, and p-alkyl styrenes. However, multi-unsaturated monomers are not precluded.

In some embodiments if desired, group E may be used to provide cross-linking. Exemplary crosslinking monomers include at least one member selected from the group consisting of [meth]acrylic esters as well as allyl and vinyl ethers of di or multifunctional alcohols, such as 1,2-ethanediol, 1,2-propanediol, 1,3-propanediol, 1,2-butanediol, 1,3-butanediol, 2,3-butanediol, 1,4-butanediol, but-2-ene-1,4-diol, 1,2-pentanediol, 1,5-pentanediol, 1,2-hexanediol, 1,6-hexanediol, 1,10-decanediol, 1,2-dodecanediol, 1,12-dodecanediol, neopentylglycol, 3-methylpentane-1,5-diol, 2,5-dimethyl-1,3-hexanediol, 2,2,4-trimethyl-1,3-pentanediol, 1,2-cyclohexanediol, 1,4-cyclohexanediol, 1,4-bis(hydroxymethyl)cyclohexane, diethyleneglycol, triethyleneglycol, tetraethyleneglycol, dipropyleneglycol, tripropyleneglycol, tetrapropyleneglycol, polyethyleneglycols, polypropyleneglycols, polytetrahydrofurans, trimethylolpropane, glycerol, pentaerythritol, 1,2,5-pentanetriol, 1,2,6-hexanetriol, and sorbitol.

Further suitable crosslinkers can include at least one of urethane diacrylates; straight chain or branched, linear or cyclic, aliphatic or aromatic hydrocarbons with at least 2 double bonds such as divinyl benzene; [meth]acrylamides and N-allyl amines of difunctional amines such as 1,2-diaminoethane, 1,3-diaminopropane, 1,4-diaminobutane, 1,6-diaminohexane; triallyl amine and triallylammonium salts, such as triallylmethylammonium chloride or methylsulfate; N-vinyl compounds of urea derivatives such as N,N'-divinylethyleneurea or N,N'-divinylpropyleneurea; di, tri, or tetra functional sulfhydryl compounds such as ethyleneglycol dimercaptopropionate, glycerol trimercaptoacetate, and pentaerythritol tetramercaptopropionate; alkoxysilane functional mercaptans such as 3-mecaptopropyl trimethoxy silane; and alkoxy silane functional alkyl [meth]acrylates or alkyl [meth]acrylamides such as methacryloxypropyl triethoxy silane and methacrylamidopropyl trimethoxy silane.

### Description of properties/attributes of copolymer

The concentration of ionic and ionogenic repeats in the ionic/ionogenic comb copolymers described herein is in the range of about 10 to about 450 meq per 100 grams of polymer, and typically about 15 to about 400 meq per 100 grams of polymer. Throughout this disclosure, the weight of cations present to neutralize anionic repeats, if present, are not included in the determination of the weight of the polymer. If zwitterionic or amphoteric monomers are included in the comb copolymer, a mole of zwitterionic or amphoteric repeat is treated as an equivalent. In some embodiments, the range of ionic and ionogenic repeats in the inventive copolymers is between about 25 and about 325 meq per 100 grams of polymer, in other embodiments is between about 25 and about 300 meq per 100 grams of polymer, and in other embodiments is between about 50 and about 275 meq per 100 grams of polymer.

The weight of associative moieties, Z, within the copolymer can be in the range of about 3 to about 40 g per 100 grams of polymer, in some embodiments is between about 5 to about 40 g per 100 grams of polymer, in other embodiments is between about 7 and about 30 g per 100 grams of polymer, and in still other embodiments is between about 10 and about 25 g per 100 grams of polymer.

Without wishing to be bound by any theory or explanation, it is believed that the ratio of associative moieties Z to the ionic and/or ionogenic repeats in the copolymer is at least partially responsible for the superior results obtained. That is, according to certain exemplary embodiments, the ionic and/or ionogenic repeats of group A, along with any other ionic moieties incorporated from groups C through E, have a relationship to the amount of associative groups Z present in the copolymer.

The ratio of associative moieties Z to ionic and/or ionogenic monomers within the copolymer can be in the range of about 0.025 to about 1.3 g/meq. In some embodiments the ratio is between about 0.040 and about 1.0 g/meq, in another case is between about 0.065 and about 0.6 g/meq, and in yet other cases is between about 0.11 and about 0.53 g/meq. In still another embodiment the ratio of associative moieties Z to ionic and/or inogenic monomers within the copolymer is about 0.20 to about 0.45 g/meq. The ionic/ionogeniccomb copolymers may be crystalline or non-crystalline. In crystalline embodiments where the copolymer exhibits a melting temperature Tₘ with a heat of fusion greater than about 3 J/g, the melting temperature of the copolymers is typically less than about 100 °C, in some cases is less than about 50 °C, in many cases is less than about 25 °C, in other cases is less than about 15 °C, and in still other cases is less than about 0 °C.

Despite its ability to thicken personal care products containing additional ingredients, in certain exemplary embodiments, a three percent (solids) weight solution of the ionic/ionogenic comb copolymer described herein h water at pH 7.0 ± 1.0 has a viscosity at 25 °C of less than about 250 cP and in some embodiments has a viscosity at 25 °C of less than about 100 cP. In still other embodiments, a three percent (solids) weight solution of the ionic/ionogeniccomb copolymer at pH 7.0 ± 1.0 has a viscosity at 25 °C of less than about 50 cP. Thus, unlike the prior art, certain exemplary embodiments do not have to rely on thickening the water phase of personal care formulations as is common with currently-used high molecular weight and frequently cross-linked polymers, while substantial sensory and deposition benefits can be achieved over prior art anionic polymer additives.

The polymer chemistry can be tailored for compatibility and solubility in various aqueous cosmetic formulations by adjusting the influence of hydroxyl, amide and other secondary monomers. The nitrogen content in the amide monomer, for example, can enhance deposition when lower ionic levels are desirable and contributes to both compatibility and viscosity. Some hydroxyl monomer content can improve polymer and formulation clarity in aqueous or alcoholic systems.

In one embodiment, ionic/ionogenic comb copolymers exhibit a range of solubility in water upon neutralization to a pH of 7.0 ± 1.0. In some embodiments the solubility in water at 25 °C is relatively low, for example, a water solubility of less than about 50 wt%, in other embodiments is less than about 20 wt%, and in still other embodiments is less than about 10 wt%. By "water solubility" or "water soluble" it is meant measuring the solubility of the copolymer in water upon neutralization to a pH of 7.0 ± 1.0 as the concentration at which the turbidity of the aqueous solution first measures 250 EBC (European Brewery Convention Standard) units, with turbidity of the aqueous solution less than 250 EBC at lower concentrations.

Advantageously, certain exemplary embodiments may also include, but unlike the prior art do not require, crosslinking of the copolymer and/or the presence of sulphonic or phosphonic acid repeats which can lead to undesirable color or odor. By "crosslinking" or "crosslinked", it is meant, the joining of adjacent chains of the copolymer by covalent bonds created through the purposeful introduction of a polyfunctional monomer. The covalent bonds that result in crosslinking may be formed through any reaction including condensation and addition reactions.

In one embodiment the ionic/ionogenic comb copolymer is comprised of about 3 to about 27 wt% of monomers of Group A, is comprised of about 25 to about 80 wt% of a monomer of Structure 2 wherein X₁ =O, n=q=r=0, p is between 3 and 30 and Z is an alkyl group having in the range of 10 to 18 carbon atoms, has between about 5 and about 30 grams of associative moieties Z per 100 grams of polymer, and in other cases has between about 7 and about 30 grams of associative moieties Z per 100 grams of polymer, and is not crosslinked. In one aspect of this embodiment, the aforementioned ionic/ionogenic comb copolymer exhibits a solution viscosity in water at three weight percent (solids) and pH 7.0 ± 1.0 of less than about 100 cP when measured at 25 °C. In one aspect of this embodiment the amount of the monomer of Structure 2 is greater than 25wt.% and typically greater than about 30wt.% (e.g., about 30wt.% to about 80wt.%).

In another embodiment the ionic/ionogenic comb copolymer is comprised of about 3 to about 27 wt% of monomers of Group A, is comprised of about 25 to about 75wt% of a monomer of Structure 2 wherein X₁=O, n=q=r=0, p is between 3 and 30 and Z is an alkyl group having in the range of 10 to 18 carbon atoms, is comprised of an acrylamide monomer of Group C, has between about 5 and about 40 grams of associative moieties Z per 100 grams of polymer, and in other cases has between about 7 and about 30 grams of associative moieties Z per 100 grams of polymer, and is not crosslinked. In one aspect of this embodiment the amount of the monomer of Structure 2 is between about 25wt.% and about 50wt.%. In one aspect of this embodiment the amount of the acrylamide monomer of Group C is between about 10wt.% and about 35wt.%. In one aspect of this embodiment the amount of the acrylamide monomer of Group C is between about 35wt.% and about 65wt.%. In one aspect of this embodiment the ionic/ionogenic comb copolymer exhibits a solution viscosity in water at three weight percent (solids)and pH 7.0 ± 1.0 of less than about 100 cP when measured at 25 °C. In one aspect of this embodiment, repeats of A are unsaturated carboxylic acid monomers or their salts, monomer of Structure 2 has p between 2-15 and comprises 30 to 75wt.% of the copolymer. In one aspect of this embodiment the amount of the monomer of Structure 2 is greater than 25wt.%, in some cases greater than about 30wt.% and typically greater than about 50wt.% (e.g., about 50wt.% to about 75wt.%).

In another embodiment the ionic/ionogenic comb copolymer is comprised of about 3 to about 27 wt% of monomers of Group A, is comprised of about 25 to about 75 wt% of a monomer of Structure 2 wherein X₁=O, n=q=r=0, p is between 3 and 30 and Z is an alkyl group having in the range of 10 to 18 carbon atoms, is comprised of a hydroxyalkyl [meth]acrylate monomer of Group D, has between about 5 and about 40 grams of associative moieties Z per 100 grams of polymer, and in other cases has between about 7 and about 30 grams of associative moieties Z per 100 grams of polymer, and is not crosslinked. In one aspect of this embodiment the amount of hydroxyalkyl[meth]acrylate monomer of Group D is between about 10wt.% and about 40wt.%. In one aspect of this embodiment the comb copolymer exhibits a solution viscosity in water at three weight percent (solids) and pH 7.0 ± 1.0 of less than about 100 cP when measured at 25 °C. In one aspect of this embodiment, wherein repeats of A are unsaturated carboxylic acid monomers or their salts, monomer of Structure 2 has p=4 to 25 and comprises 30 to 75wt.% of the copolymer and the Group D monomer is hydroxypropyl methyacrylate or hydroethyl acrylate. In one aspect of this embodiment the amount of the monomer of Structure 2 is greater than 25wt.%, in some cases greater than about 30wt.% and typically greater than about 50wt.% (e.g., about 50wt.% to about 75wt.%).

In another embodiment the ionic/ionogenic comb copolymer is comprised of about 20 to about 80 wt% of monomers of Structure 4A or Structure 4B wherein R₁ and R₂ are independently either H or CH₃, b=0, a is between 2 and 30 and Z is an alkyl group having in the range of 10 to 18 carbon atoms, has between about 5 and about 40 grams of associative moieties Z per 100 grams of polymer, and in other cases has between about 7 and about 30 grams of associative moieties Z per 100 grams of polymer, and is not crosslinked. In one aspect of this embodiment, the comb copolymer is comprised of about 20 to about 75 wt% of a monomer of Structure 4A and a is between 4 and 12. In one aspect of this embodiment the comb copolymer exhibits a solution viscosity in water at three weight percent (solids) and pH 7.0 ± 1.0 of less than about 100 cP when measured at 25 °C. In another aspect of this embodiment, the ionic/ionogenic copolymer is comprised of at least one additional monomer selected from Group A or Structure 2. In another aspect of this embodiment, the ionic/ionogenic comb copolymer is comprised of at least one additional monomer selected from an acrylamide monomer of Group C or a hydroxyalkyl[meth]acrylate monomer of Group D.

In another embodiment the ionic/ionogenic comb copolymer is comprised of about 5 to about 40 wt% of zwitterionic Group A monomers, is comprised of about 25 to about 75 wt%, and in some cases about 25 to about 50 wt%, of a monomer of Structure 2 wherein X₁=O, n=q=r=0, p is between 3 and 30 and Z is an alkyl group having in the range of 10 to 18 carbon atoms, has between about 5 and about 40 grams of associative moieties Z per 100 grams of polymer, and is not crosslinked. In one aspect of this embodiment, the comb copolymer is comprised of about 10 to about 30 wt% of zwitterionic Group A monomers. In one aspect of this embodiment the comb copolymer exhibits a solution viscosity in water at three weight percent (solids) and pH 7.0 ± 1.0 of less than about 100 cP when measured at 25 °C. In another aspect of this embodiment, the ionic/ionogenic copolymer is comprised of at least one additional monomer selected from Group C. D or E. In another aspect of this embodiment, the at least one additional monomer comprises an acrylamide monomer of Group C.

The ionic/ionogenic comb copolymer can be manufactured according to any suitable process that results in the monomers being incorporated into the desired chain structure. Polymerization may be effected in the bulk state, in solution using a suitable solvent, in emulsion, in inverse emulsion, in suspension or dispersion. One particularly suitable method of copolymer synthesis is solution polymerization in alcohol, glycol, or aqueous alcoholic mixture in the absence of any cross-linking components. The use of solution polymerization typically leads to the repeating units of the copolymer being incorporated randomly, though block, alternating or graft arrangements of repeating units are also possible. Free radical initiators may be used, and include for example peroxide, azo, and redox initiators. One or more free radical initiators are typically employed, preferably in the range of about 0.1 to about 3 weight percent based on total monomer weight. Chain transfer agents, which are well known in the polymer arts, may optionally be used during polymerization. The polymerization reaction may be carried out between about 30 and about 95 °C, and may be carried out between about 40 and about 85 °C.

In some embodiments, the copolymer is formulated into a personal care composition or product also including a cosmetically acceptable base material along with at least one active ingredient. The use of ionic/ionogeniccomb copolymer compositions describe herein can increase the deposition of the active ingredient on keratinous substrates over a like formulation in which the copolymer is not used. The deposition of the active ingredient within a composition or product is determined by application of about 1 to about 4 mg/cm² of the composition or product to a keratinous substrate such as in-vitro synthetic skin, followed by drying of the substrate for 5 to 30 minutes, then immersion of the substrate into a petri-dish with 30 mL of deionized (DI) water under agitation with a smooth magnetic stirrer set at a speed of 300 rpm and maintained at a constant temperature between room temperature (22 °C ± 2) and body temperature (37 °C ± 2) for a test period of 30 seconds to 20 minutes. Following immersion, the deposition of the active ingredient on the substrate is determined by any suitable analytical method for quantifying the active ingredient (e.g., HPLC, energy dispersive x-ray spectrometry analysis, among other suitable methods). In cases where the active ingredient has a water solubility less than 50 g/L, the deposition is increased by about 10% or more, in some cases by about 20% or more, and in other cases the deposition is increased by about 50% or more. In embodiments in which the active ingredient has a water solubility greater than 50 g/L, the use of the copolymers can increase deposition by about 10% or more, in some cases by about 20% or more, and in other cases by about 200% or more. In some cases, the deposition of the active ingredient may be increased by about 500% or more. In some embodiments the ionic/ionogenic comb copolymer compositions described herein can increase the deposition of both an active ingredient with water solubility less than about 50 g/L and an active ingredient with water solubility greater than about 50 g/L by percentages indicated above.

In still other embodiments, the use of the ionic/ionogenic comb copolymers described herein can itself demonstrate deposition to keratinous substrates.

### Personal care products and exemplary formulations using copolymer

Thus, the ionic/ionogeniccomb copolymer compositions disclosed herein may be employed as an ingredient in creating a personal care product that provides increased effectiveness in the deposition and retention of actives, has superior sensory attributes, and/or as a thickener. The ionic/ionogeniccomb copolymer composition is generally present at about 0.1 to about 20% by weight in such products, in some cases between about 0.5 and about 10% by weight, and in still other cases is present between about 1 % and about 5% by weight. The composition can be used with a wide range of personal care products having a base media that can comprise at least one member selected from the group consisting of cosmetic oil (i.e. oils compatible for cosmetic uses), water, alcohol and combinations thereof, all by way of example only.

The ionic/ionogeniccomb copolymers can be added to personal care products as pure copolymer, or as a solution or suspension in water or any suitable organic solvent or combination of solvents and/or water. When an inventive copolymer is added to personal care products in a suitable organic solvent or combination of solvents and/or water, it is generally desirable that the concentration of copolymer is at least 50 wt% if the ratio of water to solvent is less than 1:1. Alcohol and glycol are particularly suitable organic solvents. While any suitable glycol can be employed, examples of suitable glycols comprise at least one of propylene glycol, butylene glycol, and glycerol. For example, the ionic/ionogenic comb polymers can be provided as a solution in butylene glycol and water. The amount of glycol can range from about 10 to about 70wt.% of the solution, in some cases about 20 to about 60wt.% and in other cases about 30 to about 50wt.% of the solution.

Exemplary cosmetic, toiletry and topical health care type personal care products in which the ionic/ionogenic comb copolymers may be employed include, without limitation, leave-on personal care products (e.g. products that are left on keratinous substrates after application); rinse-off personal care products (e.g., products that are washed or rinsed from keratinous substrates during or within a few minutes of application); at least one of shampoos; chemical and non-chemical hair curling and hair straightening products; hair style maintaining and hair conditbning products; lotions and creams for nails, hands, feet, face, scalp and/or body; hair dye; face and body makeup; nail care products; astringents; deodorants; antiperspirants; anti-acne; anti-aging;depilatories; colognes and perfumes;skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products), any or the foregoing of which may additionally contain pharmaceutical, phytopharmaceutical and/or neutraceutical ingredients.

The form of such personal care products is not limited and may be, without limitation, a liquid, gel, spray, emulsion (such as lotions and creams), shampoo, pomade, foam, ointment, tablet, stick (such as lip care products), makeup, suppositories, among others, any of which can be applied to the skin or hair and which typically are designed to remain in contact therewith until removed, such as by rinsing with water or washing with shampoo or soap. Gels can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols. In some embodiments, exemplary ionic/ionogenic comb copolymers are formulated in aerosol compositions such as mousse, spray, or foam forming formulation, where a chemical or gaseous propellant is used.

The foregoing personal care products in which ionic/ionogenic comb copolymers in accordance with exemplary embodiments advantageously may be used generally, but not necessarily, can be broadly categorized as anhydrous oil-base products, water-in-oil emulsions, oil-in-water emulsions, or aqueous or alcohol based systems.

The ionic/ionogeniccomb copolymer composition can be effective with a broad range of cosmetic oils, such as at least one of esters (e.g., alkyl benzoates having between 12 to 15 carbons), triglycerides (e.g., Caprylic/Caprylate triglyceride), hydrocarbons (e.g., mineral oil, sunflower oil), natural oils (e.g., jojoba oil, safflower oil), and castor oil, among others. Suitable oils are also disclosed, for example, at column 3, line 37, to column 4, line 4, of U.S. Patent No. 5,736,125; hereby incorporated by reference. Silicone oils may also be used as cosmetic oils. In general, any natural or synthetic oil for cosmetic use is suitable for the composition of present invention. Non-limiting examples of natural oils are at least one of avocado oil, coconut oil, palm oil, sesame oil, peanut oil, sunflower oil, almond oil, peach kernel oil, wheat germ oil, macadamia nut oil, night primrose oil, jojoba oil, castor oil, olive oil, soya oil and the derivatives thereof. Mineral oil such as paraffin oil and petrolatum are also suitable.

Suitable synthetic oils are at least one member selected from the group consisting of fatty alcohols; fatty acid esters such as isopropyl myristate, palmitate, stearate and isostearate; oleyl oleate; isocetyl stearate; hexyl laurate; dibutyl adipate; dioctyl adiphate; myristyl myristate; oleyl erucate; polyethylene glycol and it derivatives; polyglyceryl fatty acid esters; and cetyl palmitate, by way of example only.

Silicone oils are also suitable. Useful silicone oils are non-volatile silicone oils known by INCI names that include dimethicone or dimethiconol. Volatile silicone oils such as cyclomethicones may also be used.

The following are non-limiting examples of anhydrous cosmetic formulations containing modified oils incorporating ionic/ionogenic comb copolymer compositions as described herein:
A) Thickened Anhydrous Oils suitable for personal care applications (e.g. hair gels):
   Oils - about 50 to about 95 wt%
   ionic/ionogenic comb copolymer - about 0.1 to about 20 wt%
   Complementary polymer - up to about 5 wt%
   Other Additives or Actives - about 0.1 to about 30 wt%
B) Anhydrous Scalp Serum:
   Oils - about 50 to about 95 wt%
   ionic/ionogenic comb copolymer - about 0.1 to about 20 wt%
   Complementary polymer - up to about 5 wt%
   Other Additives or Actives - about 0.1 to about 30 wt%
C) Anhydrous Sunscreen Stick or Gel:
   Oils - about 50 to about 95 wt%
   ionic/ionogenic comb copolymer - about 0.1 to about 20 wt%
   Complementary polymer - up to about 5 wt%
   Sunscreen Active - about 1 to about 25 wt%
   Other Additives or Actives - about 0.1 to about 30 wt%
D) Anhydrous Antiperspirant Deodorant Stick or Gel:
   Emollient - about 50 to about 95 wt%
   ionic/ionogenic comb copolymer - about 0.1 to about 20 wt%
   Complementary polymer - up to about 5 wt%
   Antiperspirant deodorant (APDO) actives - about 0.1 to about 30 wt%
   Other Additives or Actives - about 1 to about 30 wt%
E) Color Cosmetic (e.g. blush, lipstick)
   Oil - about 50 to about 95 wt%
   ionic/ionogenic comb copolymer - about 0.1 to about 20 wt%
   Complementary polymer - up to about 5 wt%
   Pigment - about 0.1 to about 30 wt%
   Other Additives or Actives - about 0.1 to about 10 wt %

Conventional thickeners such as waxes comprising at least one of carnauba wax, beeswax, and Candelilla wax, among others, may be employed as an additive to supplement the thickening effect of the formulations achieved by the ionic/ionogenic comb copolymer.

Water-in-oil emulsions can be prepared by mixing together (1) a heated (i.e., melted) solution of the ionic/ionogenic comb copolymer composition in any of the previously disclosed oils and (2) an aqueous phase, the aqueous phase being at a temperature similar to the oil solution (typically within about 10°C); and then cooling the mixture while stirring. Alternatively, the ionic/ionogenic comb copolymer composition could instead be initially added to the aqueous phase, or it could be added after the oil and aqueous phase has been emulsified. Regardless of the manner in which the ionic/ionogenic comb copolymer composition is added, the ratio of the aqueous phase to the oil phase can be, for example, about 0.5:1 to about 9:1.

The following are non-limiting examples of cosmetic formulations comprising water-in-oil emulsions:
A) Skin Moisturizer
   Water - about 50 to about 90 wt%
   Silicone-about 1 to about 10 wt%
   Emulsifier - about 0.5 to about 5 wt%
   Emollient - about 5 to about 20 wt%
   Ionic/ionogenic comb copolymer - about 0.1 to about 20 wt%
   Complementary polymer - up to about 5 wt%
   Other Additives or Actives - about 0.1 to about 3 wt%
B) Sunscreen
   Water - about 50 to about 90 wt%
   Silicone - about 1 to about 10 wt%
   Emulsifier - about 0.5 to about 5 wt%
   Emollient - about 5 to about 20 wt%
   Ionic/ionogenic comb copolymer - about 0.1 to about 20 wt%
   Complementary polymer - up to about 5 wt%
   Sunscreen Active - about 1 to about 25 wt%
   Other Additives or Actives - about 0.1 to about 3 wt%
C) Antiperspirant Deodorant
   Water - about 50 to about 90 wt%
   Silicone - up to about 10 wt%
   Emulsifier - about 0.5 to about 5 wt%
   Emollient - about 1 to about 20 wt%
   Ionic/ionogenic comb copolymer - about 0.1 to about 20 wt%
   Complementary polymer - up to about 5 wt%
   APDO actives - about 0.1 to about 30 wt%
   Other Additives or Actives - about 0.1 to about 5 wt%

Oil-in-water emulsions are prepared by mixing together (1) a heated (i.e., melted) solution of the ionic/ionogenic comb copolymer composition in the oil phase and (2) an aqueous phase, the aqueous phase being at a temperature similar to the emollient solution (typically within about 10°C); and then cooling the mixture while stirring. However, as with the water-in-oil emulsions, the ionic/ionogenic comb copolymer composition may initially be added to the aqueous phase or added post-emulsification. The ratio of the oil phase to the water phase can be, for example, about 0.1:1 to about 1:1. The following are non-limiting examples of cosmetic formulations comprising oil-in-water emulsions:
A) Skin Moisturizer
   Water - about 50 to about 90 wt%
   Emulsifier - about 0.5 to about 5 wt%
   Emollient - about 1 to about 20 wt%
   Ionic/ionogenic comb copolymer - about 0.1 to about 20 wt%
   Complementary polymer - up to about 5 wt%
   Other Additives or Actives - about 0.1 to about 3 wt%
B) Sunscreen
   Water - about 50 to about 90 wt%
   Emulsifier - about 0.5 to about 5 wt%
   Emollient - about 1 to about 20 wt%
   Ethanol and/or other organic solvent - up to about 35 wt%
   Ionic/ionogenic comb copolymer - about 0.1 to about 20 wt%
   Complementary polymer - up to about 5 wt%
   Sunscreen Active - about 1 to about 25 wt%
   Other Additives or Actives - about 0.1 to about 3 wt%
C) Mousse or other hair styling product
   Water - about 50 to about 90 wt%
   Emulsifier - about 0.5 to about 1 wt%
   Surfactant - about 0.1 to about 2 wt%
   Ionic/ionogenic comb copolymer - about 0.1 to about 20 wt%
   Complementary polymer - up to about 5 wt%
   Other Additives or Actives - about 0.1 to about 2 wt%
   Solvent - about 1 to about 25 wt%
   Propellant- up to about 10 wt%
D) Antiperspirant Deodorant
   Water - about 50 to about 90 wt%
   Silicone - up to about 10 wt%
   Emulsifier - about 0.5 to about 5 wt%
   Emollient - about 1 to about 20 wt%
   Ethanol and/or other organic solvent - up to about 35 wt%
   Ionic/ionogenic comb copolymer - about 0.1 to about 20 wt%
   Complementary polymer - up to about 5 wt%
   APDO actives - about 0.1 to about 30 wt%
   Other Additives or Actives - about 0.1 to about 5 wt%

The following are non-limiting examples of cosmetic formulations comprising alcohol or aqueous systems.
A) Coloring Shampoo
   Water - about 50 to about 90 wt %
   Surfactant - about 2 to about 20 wt%
   Foam booster -up to about 20 wt%
   Ionic/ionogenic comb copolymer - about 0.1 to about 20 wt%
   Complementary polymer - up to about 5 wt%
   Other Additives or Actives - about 0.1 to about 10 wt%
B) Hair Spray (aerosol and non-aerosol)
   Water - about 10 to about 90 wt%
   Ionic/ionogenic comb copolymer - about 0.1 to about 20 wt%
   Complementary polymer - up to about 5 wt%
   Ethanol or other solvents - about 33 to about 90 wt%
   Optional Propellant for an aerosol - up to about 50 wt%
   Other Additives or Actives - about 0.1 to about 2 wt%
C) Shampoo
   Water - about 50 to about 90 wt%
   Surfactant - up to about 20 wt%
   Foam booster - about 2 to about 20 wt%
   Ionic/ionogenic comb copolymer - about 0.1 to about 20 wt%
   Complementary polymer - up to about 5 wt%
   Other Additives or Actives - about 0.1 to about 10 wt%
D) Hair Styling products
   Water - about 10 to about 90 wt%
   Ionic/ionogenic comb copolymer - about 0.1 to about 20 wt%
   Complementary polymer - up to about 5 wt%
   Ethanol or other solvents- about 0 to about 10 wt%
   Other Additives or Actives - about 0.1 to about 10 wt%
E) Body cleansing products
   Water - about 50 to about 90 wt%
   Surfactant - about 2 to about 20 wt%
   Ionic/ionogenic comb copolymer - about 0.1 to about 20 wt%
   Complementary polymer - up to about 5 wt%
   Foam booster - up to about 20 wt%
   Other Additives or Actives - about 0.1 to about 10 wt%.
F) Sunscreen
   Water -up to about 10 wt%
   Emollient - about 1 to about 10 wt%
   Ethanol and/or other organic solvent - about 50 to about 95 wt%
   Ionic/ionogenic comb copolymer - about 0.1 to about 20 wt%
   Complementary polymer - up to about 5 wt%
   Sunscreen Active - about 1 to about 25 wt%
   Other Additives or Actives - about 0.1 to about 3 wt%

In products in which emollients are employed, any suitable emollient for use in cosmetic compositions can be used. Examples of suitable emollients include at least one of esters (e.g., C12-15 alkyl benzoate) and triglycerides (e.g., Caprylic/caprylate triglyceride); hydrocarbon oils (e.g., mineral oil); natural oils (e.g., Jojoba oil, safflower oil), tridecyl trimellitate, sunflower oil, castor oil, and can include aforementioned cosmetic oils, among other compounds used to impart desired or improved sensory or aesthetic properties of a personal care composition.

In products in which emulsifiers are employed, any suitable cosmetic emulsifier having a hydrophilic-lipophilic balance (HLB) in the range of about 1 to about 20 can be used. The emulsifier can be nonionic, cationic, anionic, or amphoteric or a combination of such emulsifiers can be used.

Examples of nonionic emulsifiers are at least one of laureths, e.g. laureth-4; ceteths, e.g. ceteths-1; polyethylene glycol cetyl ether; ceteareths, e.g. ceteareth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate; cetyltrimonium chloride; cetyltrimonium bromide; cocotrimonium methosulfate; as well as emulsifiers that contain a quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates; N-alkylsarcosinates; acyl taurates; acyl isethionates; alkyl phosphates; alkyl ether phosphates; alkyl ether carboxylates; alpha-olefinsulfonates, and the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Surfactants and/or foam boosters may also be employed and like the emulsifiers can be nonionic, cationic, anionic, or amphoteric or a combination of such surfactants can be used.

Suitable anionic surfactants are for example, at least one of alkyl sulfates; alkyl ether sulfates; alkylsulfonates; alkylarylsulfonates; alkyl succinates; N-alkylsarcosinates; acyl taurates; acyl isethionates; alkyl phosphates; alkyl ether phosphates; alkyl ether carboxylates; alpha olefinsulfonates, and may include the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Suitable amphoteric surfactants are, for example, at least one of alkylbetaines; alkylamidopropylbetaines; alkylsulfobetaines; alkyl glycinates; alkylcarboxyglycinates; alkyl amphoacetates or amphopropionates; and alkyl amphodiacetates or amphodipropionates. For example, it is possible to use cocodimethylsulfopropylbetaine, laurylbetaine, and cocamidopropylbetaine or sodium cocamphopropionate as surfactants.

Examples of nonionic surfactants are the reaction products of aliphatic alcohols having between 6 and 20 carbon atoms in the alkyl chains, which can be linear or branched with ethylene oxide and/or propylene oxide. Also suitable are at least one of alkylamine oxides; mono- or dialkylalkanolamides; fatty acid esters of polyethylene glycols; alkyl polyglycosides and sorbitan ether esters.

Examples of cationic surfactant are quaternary ammonium compounds, for example, cetyltrimethylammonium chloride, as well as other surfactants that contain a quaternized nitrogen.

In products in which propellant or solvents are employed, those may include at least one of isobutane, butane, dimethyl ether, and ethanol, among others.

Examples of other additive compounds include one or more members selected from the group consisting of silicone based plasticizers, natural or synthetic compounds (e.g., polysaccharides, natural or synthetic gums, stabilizers, anionic and nonionic associative thickener or rheology modifiers soluble in oil or water phase), among other compounds. The additives may include at least one compound selected from the group consisting of preservatives, stabilizers (e.g., Xanthan Gum), humectants (e.g., MP Diol, Sorbitol, and Hexylene Glycol), antioxidant (e.g., Vitamins), rheology modifiers, fragrances, and pigments, among other additives.

In some personal care products, active compounds that interact with or protect skin or hair can be included. Examples of such active compounds include at least one of sunscreen compounds (e.g. zinc oxide, titanium dioxide, octinoxate, octocrylene, ethylhexyl salicylate, oxybenzone); skin whiteners (e.g. salicylic acid); anti-cellulite compounds; anti-aging compounds (e.g., polypeptides such as Argininie/Lysine, Argininie PCA, Aspergillus/Aspidosperma Quebracho Ferment, Avena Sativa (Oat) Kernel Protein, and Avocado Sterols, proteins, peptides, copper peptides, fermented biopolymers, beta-glucan, botanical actives, Bifida Ferment Lysate, Calophylum Inorhylum seed oil, camellia sinensis extract, ceramides, chlorella vulgaris extract, coriolus versicolor extract, corylus avellana (hazel) seed extract, Hyaluronic acid, erythorbic acid, hydrolyzed elastins, hydrolyzed proteins, hydrolyzed soy flour, hydrolyzed peptides, and Vitamins A, E, C, K, and B5 as well as Niacinamide); antidandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); Polysaccharides (e.g., Hyaluronic acid, B-1,3-glucans, Chitosan); Botanicals (e.g., Aloe vera, Green tea extract, Grape seed extract, Isoflavones, Chamomille/bisabolol, Fennel, Ginko, Ginseng, Guava); Alpha Hydroxy Acids (e.g., Citric acid, Glycolic acid, Lactic acid); Sugar cane extracts; insect repellents; and Coenzymes and Enzymes (e.g., Ubiquinone, Coenzyme Q10), all by way of example only. It will be appreciated that certain active compounds may fall into more than one category and/or be used to accomplish more than one result

For purposes of this invention, silicone ols and additives are considered active ingredients. Non-limiting examples of silicone oils or additives that may be incorporated in personal care products are known by INCI names that include dimethicone, dimethiconol, siloxane and cydomethicones. In certain embodiments, the inventive ionic comb copolymer can improve deposition of actives including silicone oils.

### Discussion of optional "other polymer" in conjunction with ionic comb copolymer in personal care formulations

Personal care products may employ the ionic/ionogenic comb copolymer compositions alone as a deposition, sensory and/or thickening agent, while in other embodiments, the compositions may optionally employ up to about 5% by weight of one or more additional, complementary polymers. The complementary polymer(s) can be any type of polymer, including those which are at least one of nonionic, amphoteric or zwitterionic, anionic, cationic or mixtures of such types of polymers.

Exemplary synthetic, nonionic complementary polymers include at least one of vinyl pyrrolidone homopolymer and copolymers, including those which have a vinyl acetate group, such as, for example, those under the trade name "Luviskol" including the homopolymers Luviskol® K30, K60, K90 as well the copolymers Luviskol® VA 55 and VA 64 Plus, all available from BASF AG, in addition to Advantage® LS-E from ISP, and homopolymers of C10-30 alkyl acrylates (e.g.: Intelimer® IPA 13-1 and Intelimer IPA 13-6), all by way of example only. Natural non-ionic polymers suitable for the composition of the present invention can comprise at least one of cellulose, starches, chitosan, xanthan gum, guar gum, neutralized shellac and their derivatives.

Exemplary amphoteric polymers that may be incorporated into the personal care compositions with the ionic/ionogenic comb copolymers include at least one polymer and copolymer that are derived from acrylamides, [meth]acrylic acid, and tert-butyl amino ethylmethacrylate, such as the octylacrylamide/acrylate/butylaminoethyl [meth]acrylate copolymer available under the tradename Amphomer®; methacryloyl ethyl betaine and alkyl [meth]acrylate copolymer, such as those commercially available under the tradename Yukaformer, including Yukaformer® AM75; copolymers derived from monomers containing carboxyl groups and/or sulfonic groups (e.g. [meth]acrylic acid and itaconic acid) copolymerized with monomers such as mono or dialkylaminoalkyl[meth]acrylates or mono or diallylaminoalkyl[meth]acrylamides; as well as copolymers derived from N-octyl acrylamide, methyl [meth]acrylate, hydroxypropyl [meth]acrylate, n-tert-butylaminoethyl [meth]acrylate and/or acrylic acid, all by way of example only.

Suitable complementary anionic polymers include at least one of homopolymers and copolymers of [meth]acrylic acid or salts thereof; copolymers of [meth]acrylic acid and acrylamide or salts thereof; sodium salt of polyhydroxycarboxylic acids; water soluble or water dispersible polyester, polyurethanes (Luviset® P.U.R.) and polyureas; polyurethane acrylate combinations, including for example Hybridur® 875, and copolymers of t-butyl acrylate, ethyl acrylate, methacrylic acid (e.g. Luvimer® 100P).

Other suitable complementary anionic polymers are at least one vinyl alkyl ether copolymer, such as methyl vinyl ether/maleic acid copolymer, obtained by hydrolysis of vinyl ether/maleic anhydride copolymer and available under the trade name "Gantrez® AN or ES". These polymers may also be partially esterified, as for example, "Gantrez® ES 225" or "ES 435." Ethyl, butyl and isobutyl esters of ethyl vinyl ether/maleic acid copolymer are also useful.

Further exemplary complementary anionic polymers include at least one of Balance® CR (acrylate copolymer), Balance® 47 (Octylacrylamide/acrylate/butylaminoethyl methacrylate copolymer), Balance® 0/55 (acrylate copolymer), Aquaflex® FX 64 (ISP; isobutylene/ethylmaleimide/hydroxyethylmaleimide copolymer), Aquaflex® SF-40 (ISP; vinylpyrollidone/vinylcaprolactam/dimethylaminopropylamine acrylate copolymer), Alliance® LT-120 (ISP/Rohm & Hass; acrylate/C1-2 succinate/hydroxyacrylate copolymer), Aquarez® HS (Eastman; polyester-1), Diaformer® Z-400 (Clariant; methacryloylethylbetaine/methacrylate copolymer), Diaformer® Z-712 or Z-711 (Clariant; methacryloylethyl N-oxide/methacrylate copolymer), Intelimer 8600 Emulsion Polymer (C8-22 alkyl acrylates/methacrylic acid crosspolymer), Omnirez® 200 (ISP; monoethyl ester of poly(methyl vinylether/maleic acid), Amphomer® HC (acrylate/octylacrylamide copolymer) Amphomer® 28-4910 (octylacrylamide/acrylate/butylaminoethyl methacrylate copolymer), Advantage® HC 37 (ISP terpolymer of vinylcaprolactam/vinylpyrrolidone/dimethylaminoetyl methacrylate), Acudyne® 258(Rohm & Haas; acrylate/hydroxyl ester acrylate copolymer), Luviset® PUR (BASF, polyurethane-1), and Eastman® A48 (Eastman).

Still further useful complementary anionic polymers are at least one vinyl acetate/crotonic acid or vinyl acetate/vinyl neodecanoate/crotonic acid copolymers available under the trade name "Resyn®"; sodium acrylate/vinyl alcohol copolymer available under the trade nane "Hydagen® F"; sodium polystyrene sulfonate, e.g. "Flexan® 140"; ethyl acrylate/acrylic acid/N-tert-butyl acrylamide copolymers available under the trade name "Ultrahold®"; vinyl pyrrolidone/vinyl acetate/itaconic acid copolymer; and acrylic acid/acrylamide copolymer or the sodium salts thereof available under the trade name "Reten®;" acrylate copolymer available under trade name Salcare® SC 81; PEG/PPG 25/25 dimethicone/acrylate copolymer available under the trade name Luviflex Silk from BASF; acrylate/t-butylacrylamide copolymer available under the trade name Ultrahold Strong; vinyl caprolactam/PVP/dimethylaminoethylmethacrylate copolymer available as Advantage LC-E; and vinyl acetate/crotonates copolymer available under trade name Luviset® C 66, all by way of example only.

Complementary cationic polymers that may be used in personal care products in addition to the ionic/ionogeniccomb copolymers described herein include the cationic cellulose type polymer available under the trade name JR from Amerchol®, such as polyquaternium 10 and cationic guar gums, such as guar hydroxypropyltrimonium chloride, including those available under the tradename Jaguar®. Furthermore, chitosan and chitin can also be included as cationic natural polymers, along with cationic derivatives of natural polymers such as starches, celluloses, and xanthan gum.

Other suitable cationic polymers include any of the polyquaternium polymers, such as polyquaternium 6, polyquaternium 7, polyquaternium 11, polyquaternium 16, polyquaternium 22, polyquaternium 24, polyquaternium 28, polyquaternium 30, polyquaternium 36, polyquaternium 37, polyquaternium 46, polyquaternium 67 and polyquaternium 72, polyquaternium 101, and mixtures thereof, all by way of example only.

Other exemplary complementary cationic polymers include at least one of salts of vinylpyrrolidone/N-vinylimidazolium copolymer (e.g., Luviquat® FC, Luviquat HM, Luviquat MS, and Luviquat Care); N-vinylpyrrolidone/dimethylaminoethyl methacrylate copolymer quaternized with diethyl sulfate (e.g., Luviquat PQ-11); salts of N-vinylcaprolactum N-vinylpyrrolidone/N-vinylimidazolium copolymer (e.g., Luviquat Hold); cationic cellulose derivatives (e.g., polyquaternium-4 and -10); and acrylamide and dimethyldiallylammonium chloride copolymer (e.g., polyquaternium-7).

The complementary polymer may also include silicone compounds, such as, for example, at least one of polydiorganosiloxanes, polydialkylsiloxanes, polyalkylsiloxanes, polyarylsiloxane, silicone resins, silicone gums or dimethicone copolyols and aminofunctional silicone compounds such as amodimethicone. Other silicone compounds include graft polymers of organosiloxane and polyethyloxazolines compounds known with the INCI name Polysilicone-9. Any polymeric compound having an INCI name including silicone, methicone, dimethicone, or siloxane as part of its name may be used.

### EXAMPLES

The invention is further described by way of the following examples, which are presented by way of illustration, not of limitation of the claims attached hereto.

In the following experimental examples of ionic/ionogenic comb copolymers, Examples 1 through 20, various combinations of up to five out of a total of fourteen acrylic monomers (designated below as M1 to M14) selected from both hydrophilic and hydrophobic esters, amides, alcohols, acids, acid salts, and zwitterions were used in forming ionic/ionogenic comb copolymer compositions in accordance with exemplary embodiments.

In the examples, the composition M1 refers to the monomer BX-CSEM-25/80, a mixture of approximately 75% wt. cetyl/stearyl polyethoxy (25) methacrylate, about 5% wt. methacrylic acid and about 20% wt. water, commercially available from Bimax, Inc. M1 was used to provide group B repeating units. Weights of M1 used in examples were based on the as-supplied monomer BX-CSEM-25/80.

M2 is a group E monomer and refers to n-Butyl acrylate, available from various commercial sources at 99% purity.

M3 refers to the monomer CD9075, a mixed commercial grade of approximately 98% wt. lauryl polyethoxy (4) acrylate with about 2% wt. lauryl alcohol and ethoxylates of lauryl alcohol available from Sartomer Co; M3 was used to provide group B repeating units.

M4 refers to the monomer diacetone acrylamide, commercially obtained at 98% purity from Kyowa Hakko Chemical Co. M4 was used to provide group C repeating units.

M5 refers to the monomer dimethyl acrylamide, commercially obtained at 98% purity from Aldrich Chemical Company. M5 was used to provide group C repeating units.

M6 refers to the monomer HPMA 97, a 97-98% purity mixture of approximately 75% wt. hydroxypropyl and 25% wt. hydroxyisopropyl methacrylates commercially obtained from Aldrich Chemical Company. M6 was used to provide group D repeating units.

M7 refers to the monomer methacrylic acid, obtained from various commercial sources at 99+% purity. M7 was used to provide group A repeating units.

M8 refers to the monomer acrylic acid, obtained from various commercial sources at 99+% purity. M8 was used to provide group A repeating units.

M9 refers to the monomer sodium methacrylate, available from various commercial sources at 99+% purity. M9 was used to provide group A repeating units.

M10 refers to the monomer ethyl acrylate, available from various commercial sources at 99+% purity. M10 is used to provide group E repeating units.

M11 refers to a maleic acid - half ester of ethoxylated alcohol that is formed by reaction of equimolar amounts of maleic anhydride and a five-mole ethoxylate of linear primary alcohol with carbon number 12-13, (e.g., see also Example 1 of US 3,657,175; hereby incorporated by reference). Preparation of M11 is described in the Monomer Example herein. M11 was used to provide both group A and group B repeating units.

M12 refers to the monomer N-(3-Sulfopropyl)-N-(methacryloyloxyethyl)-N,N-dimethylammonium betaine, that was obtained from Aldrich Chemical Company. M12 was used to provide group A repeat units.

M13 refers to the monomer hydroxyisopropyl acrylate, commercially obtained from Aldrich Chemical Company. M13 was used to provide group D repeating units.

M14 refers to the monomer 2-acrylamido-2-methyl-1-propane sulfonic acid, commercially obtained from Aldrich Chemical Company. M14 was used to provide group A repeating units.

The initiator in each experimental example of ionic/ionogenic comb copolymer was selected from 4,4'-azobis[4-cyanovaleric acid], 2,2'-azobis-2-methylpriopionamidine dihydrochloride, and benzoyl peroxide, all commercially obtained from Aldrich Chemical Company.

### Monomer Example: Preparation of M11

A 1-liter jacketed reactor was equipped with an overhead mechanical stirrer, a thermocouple, and a nitrogen purge. Tomadol® 23-5 Surfactant (80.8 g, 196 mmol), a five-mole ethoxylate of linear primary alcohol with carbon number 12-13, was charged to the reactor and stirred at room temperature under nitrogen. Maleic anhydride (19.2 g, 196 mmol) and a polymerization inhibitor 4-methoxyphenol (monomethylether of hydroquinone, MEHQ) 0.01 g were added to the reactor as powders. The mixture was slowly heated to 60 °C, and maintained at 60 °C for 2 hours. The reaction product was then cooled to room temperature. Formation of the desired product M11 was confirmed by NMR analysis according to the following procedure. The M11 reaction product was dissolved in chloroform-d with chromium acetylacetonate added as a relaxation agent. The NMR experiments were performed at ambient temperature employing the Bruker Avance III 500 FT-NMR spectrometer equipped with a 10 mm BBO probe. Quantitative 13C NMR data was acquired using inverse-gated decoupling, a 30° pulse, and a 6 second relaxation delay. The chemical shift scale was referenced to the solvent peak.

### Experimental examples of ionic/ionogenic comb polymers

### Example 1

A 1-liter jacketed reactor was equipped with an overhead mechanical stirrer, a nitrogen sparge, a thermocouple, and an overhead condenser. The reactor jacket was connected to a circulating oil bath capable of heating and cooling.

A monomer mixture was prepared by stirring together 225.1 grams of M3, 75.0 grams of M7, 24.1 grams of water, 153.0 grams of 2-propanol (IPA), and 123.2 grams of 1,3-butylene glycol. About 20 wt%, 119.9 grams of the monomer mixture was charged to the reactor and sparged with nitrogen. During sparging, the reactor contents were heated to 50 °C.

The remaining 80 wt% of the monomer mixture was poured into a 500 ml Pyrex addition funnel for delayed addition to the reactor. The monomer mixture in the monomer addition funnel was maintained at a temperature of 35 to 40 °C.

An initiator mixture was prepared, consisting of 4.14 grams of 2,2'-azobis-2-methylpriopionamidine dihydrochloride dissolved in 19.84 grams of DI water and 19.84 grams of methanol.

After the initial monomer mixture charge in the reactor was sparged with nitrogen for 30 minutes, and the reactor temperature reached 50 °C, a first quantity of 7.78 grams of initiator mixture was charged to the reactor. An additional 30.97 grams of initiator mixture was added to a 30 mL syringe barrel for delayed addition to the reactor.

15 minutes after the first quantity of initiator mixture was charged to the reactor, the reactor temperature was raised to 65 °C, and the delayed monomer feed was fed into the reactor over a 60 minute period. 15 minutes after the start of the delayed monomer feed, the initiator delayed feed was fed into the reactor over a 90 minute period.

During the delayed monomer and initiator feeds, the reactor temperature was maintained at 70 to 75 °C. After all the initiator mixture was delivered, the reactor was held at 70 to 75 °C for an additional 2 hours, and subsequently the reactor temperature was lowered to 65 °C. A chase step was initiated by addition of 3.38 grams of t-butyl hydroperoxide (70 wt% in water) to the reactor; then 1.62 grams of the monosodium salt of hydroxy methanesulfinic acid dihydrate with 16.04 grams of water was prepared and delivered to the reactor over a 20 minute period, followed by an additional 30 minutes hold at 65 to 70 °C.

A nitrogen sparge and partial vacuum was applied to remove volatile solvents over a period of 4.5 hours at a temperature of 65 to 85 °C. After cooling to room temperature, a nearly clear viscous polymer solution was obtained.

### Example 2

A 1-liter jacketed reactor was equipped with an overhead mechanical stirrer, a nitrogen sparge, a thermocouple, and an overhead condenser. The reactor jacket was connected to a circulating oil bath capable of heating and cooling.

A monomer mixture was prepared bystirring together 210.0 grams M3, 69.2 grams M5, and 21.0 grams M7 with 270.2 grams IPA and 30.1 grams deionized water. About 20 wt%, 119.8 grams of the monomer mixture was charged to the reactor and sparged with nitrogen. During sparging, the reactor contents were heated to 65 °C.

The remaining 80 wt% of the monomer mixture was poured into a 500 ml Pyrex addition funnel for delayed addition to the reactor. The monomer mixture in the monomer addition funnel was maintained at a temperature of 35 to 40 °C.

An initiator mixture was prepared, consisting of 3.96 grams of 2,2'-azobis-2-methylpriopionamidine dihydrochloride dissolved in 16.63 grams of DI water and 16.63 grams of methanol.

After the initial monomer mixture charge in the reactor was sparged with nitrogen for 30 minutes, and the reactor temperature reached 50 °C, a first quantity of 6.76 grams of initiator mixture was charged to the reactor. An additional 27.53 grams of initiator mixture was added to a 30 mL syringe barrel for delayed addition to the reactor.

15 minutes after the first quantity of initiator mixture was charged to the reactor, the reactor temperature was raised to 75 °C, and the delayed monomer feed and the initiator delayed feed were fed into the reactor over 60 minute and 90 minute periods, respectively. After the initiator feed was complete, the reactor temperature was maintained at 75 °C for an additional 2 hours.

A nitrogen sparge and partial vacuum was applied to remove volatile solvents over a period of 2.5 hours at a temperature of 65 to 85 °C. After cooling to room temperature, a golden slightly opaque viscous polymer was obtained.

### Example 3

A monomer mixture was prepared bystirring together 45 grams M 1, 45 grams M3, 72 grams M5, 102 grams M6, and 36 grams M7 with 150 grams of IPA, 30 grams of deionized water and 120 grams of 1,3-butanediol. The polymer was prepared following Example 2, and a clear golden viscous solution was obtained.

### Example 4

A monomer mixture was prepared by stirring together 105 grams M1, 105 grams M3, 30 grams M8, and 60 grams M13 with 150 grams of IPA, 30 grams of deionized water and 120 grams of 1,3-butanediol. The polymer was prepared following Example 2, and a light beige slightly opaque viscous solution was obtained.

### Example 5

A monomer mixture was prepared bystirring together 105 grams M1, 105 grams M3, 60 grams M6, and 30 grams M8 with 150 grams of IPA, 30 grams of deionized water and 120 grams of 1,3-butanediol. The polymer was prepared following Example 2, and a light yellow slightly opaque viscous solution was obtained .

### Example 6

A monomer mixture was prepared bystirring together 282 grams M1 and 18 grams M7 with 150 grams of IPA, 30 grams of deionized water and 120 grams of 1,3-butanediol. The polymer was prepared following Example 2, and a light beige slightly opaque viscous solution was obtained.

### Example 7

The polymer was prepared following Example 4, except glycerol was used in place of 1,3-butanediol. A hazy yellow viscous solution was obtained.

### Example 8

A monomer mixture was prepared bystirring together 60 grams M1, 150 grams M3, 81 grams M6, and 9 grams M8 with 150 grams of IPA, 30 grams of deionized water and 120 grams of 1,3-butanediol. The polymer was prepared following Example 2, and a light yellow hazy viscous solution was obtained.

### Example 9

A monomer mixture was prepared bystirring together 195 grams M3, 60 grams M4, 36 grams M6, and 9 grams M9 with 150 grams of IPA, 30 grams of deionized water and 120 grams of 1,3-butanediol. The polymer was prepared following Example 2, and a light yellow hazy viscous solution was obtained.

### Example 10

A monomer mixture was prepared bystirring together 210 grams M3, 45 grams M4, 33 grams M6, and 12 grams M7 with 150 grams of IPA, 30 grams of deionized water and 120 grams of 1,3-butanediol. The polymer was prepared following Example 2, and a light yellow hazy viscous solution was obtained.

### Example 11

A monomer mixture was prepared bystirring together 60 grams M1, 159 grams M3, and 81 grams M8 with 150 grams of IPA, 30 grams of deionized water and 120 grams of 1,3-butanediol. The polymer was prepared following Example 2, and a clear beige viscous solution was obtained.

### Example 12

A monomer mixture was prepared bystirring together 126 grams M1, 45 grams M4, 84 grams M5, and 45 grams M12 with 150 grams of IPA, 30 grams of deionized water and 120 grams of 1,3-butanediol. The polymer was prepared following Example 2, and a light yellow hazy viscous solution was obtained.

### Example 13

A monomer mixture was prepared bystirring together 210 grams M3, 45 grams M5, and 45 grams M14 with 150 grams of IPA, 30 grams of deionized water and 120 grams of 1,3-butanediol. The polymer was prepared following Example 2, and a clear light green solution was obtained.

### Example 14

A monomer mixture was prepared bystirring together 75 grams M2, 120 grams M3, 75 grams M5, and 30 grams M9 with 150 grams of IPA, 30 grams of deionized water and 120 grams of 1,3-butanediol. The polymer was prepared following Example 2, and a translucent yellow-green viscous solution was obtained.

### Example 15

A monomer mixture was prepared bystirring together 150 grams M3, 60 grams M5, and 90 grams M12 with 150 grams of IPA, 30 grams of deionized water and 120 grams of 1,3-butanediol. The polymer was prepared following Example 2, and a white opaque viscous solution was obtained.

### Example 16

The polymer was prepared following Example 12, except 4,4'-azobis[4-cyanovaleric acid]was used in place of 2,2'-azobis-2-methylpriopionamidine dihydrochloride. An off-white opaque viscous solution was obtained.

### Example 17

The polymer was prepared following Example 14, except 4,4'-azobis[4-cyanovaleric acid] was used in place of 2,2'-azobis-2-methylpriopionamidine dihydrochloride. A clear nearly colorless viscous solution was obtained.

### Example 18

A monomer mixture was prepared by stirring together 225 grams of M3 and 75 of grams M7 with 150 grams of IPA, 30 grams of deionized water and 120 grams of 1,3-butanediol. An initiator mixture was prepared, consisting of 3.96 grams of 4,4'-azobis[4-cyanovaleric acid] dissolved in 16.63 grams of DI water and 16.63 grams of methanol. The polymer was prepared following Example 2, and an off-white opaque viscous solution was obtained.

### Example 19

A monomer mixture was prepared by stirring together 225 grams of M3 and 75 of grams M7 with 150 grams of IPA, 30 grams of deionized water and 120 grams of 1,3-butanediol. An initiator mixture was prepared, consisting of 3.96 grams of benzoyl peroxide dissolved in 32.0 grams of methyl isobutyl ketone. The polymer was prepared following Example 2, and a white opaque viscous solution was obtained.

### Example 20

A monomer mixture was prepared by stirring together 90 grams M3, 75 grams M5, 75 grams M6, and 60 grams M11 with 150 grams of IPA, 30 grams of deionized water and 120 grams of 1,3-butanediol. The polymer was prepared following Example 2, and a clear yellow viscous solution was obtained.

For each of the 20 polymer examples, the weight of associative Z moieties per 100 g of the total weight of solids in the copolymer was calculated, as was the concentration of ionogenic and ionic units, based on the particular mixture of starting monomers employed. In the examples, the associative Z moieties in each case are alkyl groups having eight or more carbon atoms (i.e., which are derived from monomers M1, M3 and M11), and the total of ionic and ionogenicunits are those which contain acid or neutralized acid or zwitterionic groups (i.e., repeating units derived from monomers M7, M8, M9, M11, M12 and M14). The ratio of associative Z moieties to the concentration of ionogenic and ionic groups was alsocalculated. Table 1 includes the calculated weight of associative Z moieties, the total of ionic and ionogenic units, and the ratio of associative Z moieties to the concentration of ionogenic and ionic groups for each exemplary ionic/ionogenic comb copolymer.

For each of the ionic/ionogenic comb copolymers of Table 1 certain measurements were determined for the samples, including the viscosity of the final polymer solution at 21 ± 1°C as reported in Table 1 and measured using a Brookfield LVTD Viscometer at 6 rpm shear rate with an LV spindle that yielded a torque reading within the recommended range for the instrument, the appearance and viscosity of the copolymer sample in DI water at pH 7.0 ± 1.0 (at 3 wt% solids copolymer) as measured using a Brookfield LVTD Viscometer at 60 rpm with UL adapter and UL spindle and reported in Table 17, along with the temperature (in °C) of any endothermic thermal peak with a heat of fusion greater than 3 J/g as measured by differential scanning calorimetry on the final polymer after removal of water or solvent (100 wt% solids) as reported in Table 1. Differential scanning calorimetry was performed on a TA Instruments Q200, calibrated in T1 mode with indium, with a sample size of approximately 10mg; the sample was heated from -90 °C to 200 °C at 10 °C/min, cooled to -90 °C at 10 °C/min, and heated again from -90 °Cto 200 °C at 10 °C/min, with the reported endothermic thermal peak obtained from the second heating. Where dashes appear in the thermal peak column of Table 1, no thermal peak was observed that met the stated criteria; where vacancies appear in Table 1, no measurement was obtained.

**Table 1**

| **Polymer Example Number** | **Associative Z [g per 100 g polymer] (calc)** | **Ionic + Ionogenic [meq per 100 g polymer] (calc)** | **Ratio Associative Z to Ionic + Ionogenic [g/meq]** | **Viscosity @ 21 ± 1 °C, Pa·s** | **Thermal Peak [°C]** |
|---|---|---|---|---|---|
| 1 | 31.7 | 290 | 0.11 | | -19.4 |
| 2 | 29.6 | 81.3 | 0.36 | 11.8 | -23.2 |
| 3 | 8.71 | 152 | 0.06 | | -- |
| 4 | 21.1 | 172 | 0.12 | 5.6 | -2.1 |
| 5 | 21.1 | 172 | 0.12 | 14.0 | -12.7 |
| 6 | 16.1 | 153 | 0.11 | | 32.9 |
| 7 | 21.1 | 172 | 0.12 | 101 | -0.6 |
| 8 | 24.9 | 56.5 | 0.44 | 11.2 | -3.3 |
| 9 | 27.7 | 27.9 | 0.99 | 12.2 | -28.0 |
| 10 | 29.6 | 46.5 | 0.64 | 8.7 | -19.8 |
| 11 | 26.3 | 402 | 0.07 | 19.0 | -21.6 |
| 12 | 6.39 | 85.4 | 0.07 | | 6.1 |
| 13 | 29.6 | 72.4 | 0.41 | 4.8 | -14.8 |
| 14 | 17.3 | 94.5 | 0.18 | | -- |
| 15 | 21.2 | 107 | 0.20 | | -7.0 |
| 16 | 6.39 | 85.4 | 0.07 | | -6.5 |
| 17 | 17.3 | 94.5 | 0.18 | | -29.1 |
| 18 | 31.7 | 290 | 0.11 | | -- |
| 19 | 31.7 | 290 | 0.11 | | -8.3 |
| 20 | 19.6 | 39.1 | 0.50 | | -30.3 |

### Discussion of exemplary formulations incorporating sample copolymers

Ionic/ionogenic comb copolymers obtained in examples 1 through 20 were separately added as an ingredient to one or more of eight different personal care products formulations as further described below, designated as series A, B, D, and M through Q. Within the formulations of series A, B, D, and M through Q, the indicated amount of ionic/ionogenic comb copolymer was added on a 100% polymer basis. These formulations were prepared as representative of common personal care product and/or to assist in analysis of features attainable through the use of the copolymers when compared to a control formulation.

Ionic/ionogenic comb copolymers obtained in examples 1 through 20 are separately added as an ingredient to nine different personal care products formulations as further described below, designated as series C, and E through L. Within the formulations of series C, and E through L, the indicated amount of ionic/ionogenic comb copolymer is added on a 100% polymer basis.

### Examples 21-40

The A series formulation was designed as representative of a low pH formulation as might be found, for example, in facial cleansers. This formulation had the total composition reflected in Table 2.

**Table 2 - Low pH formulation (A series)**

| Ingredients | % weight |
|---|---|
| **Phase A** | |
| Caprylic/caprylate triglyceride | 13.0 |
| Cetearyl alcohol | 2.0 |
| Ceteareth 20 | 4.0 |
| Glyceryl Stearate | 3.0 |

| **Phase B** | |
|---|---|
| Water | balance |
| Salicylic acid | 2.0 |
| Glycerin | 3.0 |
| Ionic/ionogenic comb copolymer | 1.0 |
| DMDM Hydantoin | 0.5 |
| **Total** | 100.0 |

Each of the ionic/ionogenic comb copolymers in examples 1 to 20 was used in creating a series A formulation, the creation followed the same following procedure. First, all of phase A ingredients were added together in a vessel and heated to 80°C. Deionized (DI) water was added to a separate phase B vessel and the sample polymer was added and neutralized with triethanolamine (TEA) to pH 7.0 ± 1.0 if needed (i.e., if the pH was below 6.0) while mixing under heat In a step wise manner, glycerin was added to the phase B vessel, and then salicylic acid until the mixture had reached 80°C and the salicylic acid was fully dissolved. The phase A mixture was added to phase B, heat was removed, and the mixture was allowed to cool. DMDM Hydantoin was added when the temperature descended below 60°C. The formulation was continuously mixed until it had cooled to room temperature.

The A series formulations were then studied to determine the effectiveness of deposition of the active ingredient, salicylic acid, using in-vitro synthetic skin. Tests were conducted by applying and rubbing 2 mg/cm² of the A series formulation for 30 seconds on the skin. After application, the skin was left to dry for 15 minutes, then immersed in a petri-dish with 30 mL DI water under agitation with a smooth magnetic stirrer set at a speed of 300 rpm. Water temperature was maintained at room temperature (22 °C ± 2).

At 1, 2, and 15 minutes after immersion, samples of the water were removed and filtered through a 5 micron filter. These samples were subsequently subjected to high performance liquid chromatography (HPLC) to analyze the amount of salicylic acid extracted with the sample and thus permit calculation of the amount of salicylic acid fiat was deposited as the difference between the amount of salicylic acid initially applied to the in-vitro synthetic skin and the amount of salicylic acid extracted, which was then averaged across the various time intervals at which samples were collected and reported in Table 17.

### Examples 41-60

The series B formulation was designed as representative of a skin cream formulation, except that a higher percentage of water than ordinarily would be present was included to study sensory feel imparted by the ionic/ionogenic comb copolymer compositions. The B series formulation had the total composition reflected in Table 3.

**Table 3 - Skin Cream (B series)**

| Ingredients | % weight |
|---|---|
| **Phase A** | |
| DI water | balance |
| Ionic/ionogenic comb copolymer | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

For each case in which one of the ionic/ionogenic comb copolymers formed in examples 1 to 20 was used in creating a series B formulation, the creation followed the same following procedure. First, all of the phase B ingredient were added together in a vessel and were mixed and heated to 80°C. The DI water and ionic/ionogenic comb copolymer were addedto a separate vessel, neutralized with TEA to pH 7.0 ± 1.0 if needed (i.e., if the pH was below 6.0), and also heated and mixed to 80°C, at which point phase B was added b Phase A. The mixture was allowed to cool to 65°C and was homogenized at 5000 rpm for 3 min. The formulation was mixed until it reached room temperature and was pH adjusted to about 5.5.

The B series formulations were evaluated for sensory characteristics by an expert sensory panel according to the following protocol.

An unwetted back of the hand and inside of the forearm were used in this study, to which 0.2-0.4 grams of the B series formulation was dabbed with one finger, and with another finger, the same amount was applied of a comparative commercial product used as a positive control and which contained 2.5% by weight dimethicone.

The sample and control formulations were applied on the forearm or back of the hand simultaneously at a distance apart from each other of approximately 2 cm. Both were rubbed in at the same time using circular and front-and-back movements of the fingers. Application of the formulations required 1-2 minutes.

Properties of each sample were evaluated for feel, drag, slip and tack/stickiness as compared to the commercial product. Feel was the perception of a nice feel substance, typically characterized from a desirable light silky feel to an undesirable heavy greasy feel. Drag was the perception of resistance encountered when moving fingers across the skin to which the formulations had been applied, particularly as the formulation reached the near-dry and dry stages. Slip was the perception of a smooth film with slight to no hesitation when moving the finger across the skin over the area where the formulations had been applied, particularly as the formulation was wet, and tack was the perception of adhesive quality of the product to the skin during and after the rubbing process.

Baseline assessment and rating criteria were established priorto the start of the study based on two control formulations: a negative control (the B series formulation of Table 3 minus the ionic/ionogeniccomb copolymer), and a positive control (Aveeno® "Active Naturals™ Skin Relief Moisturizing Lotion" available from Johnson & Johnson Consumer Companies, Inc.). Non-smoking panelists with normal to dry skin, male and female between ages 35 to 60 were used in this study. Panelists were asked not to use any other product on the hand and arm before the study. Panelists were trained in sensory attributes, and the same evaluators were used throughout the entirety of the sensory study. Panelists were asked to rate subjectively each of the experimental formulations relative to the commercial formulation. A grading scale of 1 to 5, with 1 being "very poor relative to the commercial product and same as the negative control," 4 denoting "same as the positive control commercial product," and 5 being "excellent relative to the commercial product" was used to assess the formulations, with the average sensory rating of panelists included in Table 17.

### Examples 61-80

The C series formulation is designed as representative of a facial cream formulation and has the total composition reflected Table 4.

**Table 4 - Facial cream (C series)**

| Ingredients | % weight |
|---|---|
| **Phase A** | |
| DI Water | balance |
| Glycerin | 4.0 |
| DMDM Hydantoin | 0.5 |
| Ionic/ionogenic comb copolymer | 1.0 |

| **Phase B** | |
|---|---|
| Isopropyl Palmitate | 6.0 |
| C12-15 Alkyl Benzoate | 5.0 |
| PEG-100 Stearate | 2.5 |
| Glyceryl Stearate | 2.5 |
| Cetearyl Alcohol | 2.0 |
| Dimethicone | 2.5 |
| **Total** | 100.0 |

Each of the ionic/ionogenic comb copolymers in examples 1 to 20 is used in creating a series C formulation, the creation follows the same following procedure. First, the phase B ingredients are added together in a vessel and are mixed while heating to 80°C. In a separate vessel, DI water and the sample ionic/ionogeniccomb copolymer are added while mixing, are neutralized with TEA to pH 7.0 ± 1.0 if needed (i.e., if the pH is below 6.0), and heating begins. To this mixture, the glycerin is then added. After both phases reach 80°C, the phase B mixture is added into the phase A mixture and heat is removed. The DMDM hydantoin is added after the temperature descends below 60°C, and the formulation is continuously mixed until it reaches room temperature, at which point the pH is adjusted using triethanolamine to between about 5.0 and 5.

### Examples 81-100

The D series formulation was designed as representative of a hair conditioner formulation and had the total composition reflected in Table 5.

**Table 5 - Hair conditioner (D series)**

| Ingredients | % weight |
|---|---|
| **Phase A** | |
| DI water | balance |
| Ionic/ionogenic comb copolymer | 1.0 |
| Dimethiconol (and) TEA-Dodecylbenzenesulfonate | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

Each of the ionic/ionogenic comb copolymers in examples 1 to 20 was used in creating a D series formulation, the creation followed the same following procedure. First, all of the phase B ingredients were added together in a vessel and heated under mixing to 80°C. Dl water and the ionic/ionogenic comb copolymer was added b a separate vessel, neutralized with TEA to pH 7.0 ± 1.0 if needed (i.e., if the pH was below 6.0), and heated under mixing. Next, the dimethiconol and TEA-Dodecylbenzenesulfonate were added to the phase A vessel. After both phases reached 80°C, the phase B mixture was added to the phase A mixture, at which point heat was removed. When the composition reached 65°C, it was homogenized at 5000 rpm for 3 min, followed by continual mixing until it cooled to room temperature.

The series D formulation was tested for effectiveness of the deposition of silicone as an active ingredient (present as dimethiconol). The silicone deposition studies were conducted using in-vitro synthetic skin to which 2 mg/cm² of the formulation was applied and rubbed in for 30 seconds. After application, the skin was left to dry for 15 minutes. The skin was then immersed in a Petri-dish with 30 mL DI water under agitation with a smooth magnetic stirrer having a speed of 300 rpm. Water temperature was maintained at room temperature (22 °C ± 2).

At 1, 2 and 15 minutes after immersion, samples of the water were removed and filtered through a 5 micron filter. These samples were subsequently subjected to inductively coupled plasma atomic emission spectroscopy (ICP-OES) using a Perkin-Elmer Optima 5000 dual view emission spectrometer with axial viewing with background correction and in accordance with known procedures. Results for silicon were confirmed using three analytical wavelengths: 212.412 nm, 251.611 nm and 288.158 nm. Based on the ICP silicone results for each extract, the percentage of silicone deposited on the skin was calculated as the difference between the amount of silicone initially applied to the in-vitro synthetic skin and the amount of silicone extracted, and was then averaged across the various time intervals at which samples were collected, with averaged values for percentage of silicone deposited across the various time intervals reported in Table 17.

### Examples 101-120

The E series formulation is designed as representative of a skin cream formulation and has the total composition reflected in Table 6.

**Table 6 - Skin cream (E series)**

| Ingredients | % weight |
|---|---|
| **Phase A** | |
| Mineral Oil | 8.0 |
| PPG-15 stearyl ether | 1.0 |
| Steareth-21 | 2.5 |
| Steareth-2 | 1.1 |
| Cetearyl Alcohol | 4.0 |
| Dimethiconol (and) TEA-Dodecylbenzenesulfonate | 2.5 |

| **Phase B** | |
|---|---|
| DI water | Balance |
| Glycerine | 3.0 |
| Propylene Glycol | 1.0 |
| Ionic/ionogenic comb copolymer | 1.0 |
| DMDM Hydantoin | 0.5 |
| **Total** | 100.0 |

Each of the ionic/ionogenic comb copolymers in examples 1 to 20 is used in creating a series E formulation, the creation follows the same following procedure. DI water is added to a vessel along with the ionic/ionogenic comb copolymer and mixing under heating is initiated; the mixture is neutralized with TEA to pH 7.0 ± 1.0 if needed (i.e., if the pH is below 6.0). Glycerin and propylene glycol are then added to the vessel. In a separate vessel, all of the phase A ingredients are added together and are mixed under heating. After both phases are heated to 80°C, the vessel containing the phase A materials is added to the vessel containing the phase B materials and heat is removed. DMDM hydantoin is added after the temperature descends below 60°C and the formulation is continuously mixed until it reaches room temperature, at which point the pH is adjusted using triethanolamine to between about 5.0 and 5.5.

All of the series C and E formulations are tested for effectiveness of the deposition of silicone as an active ingredient (present as dimethicone or dimethiconol). The silicone deposition studies are conducted using in-vitro synthetic skin to which 2 mg/cm² of the formulation is applied and rubbed in for 30 seconds. After application, the skin is left to dry for 15 minutes. The skin is then immersed in a Petri-dish with 30 mL DI water under agitation with a smooth magnetic stirrer having a speed of 300 rpm. Water temperature is maintained at room temperature (22 °C ± 2).

At 1, 2 and 15 minutes after immersion, samples of the water are removed and are filtered through a 5 micron filter. These samples are subsequently subjected to inductively coupled plasma atomic emission spectroscopy (ICP-OES) using a Perkin-Elmer Optima 5000 dual view emission spectrometer with axial viewing with background correction. Results for silicon are confirmed using three analytical wavelengths: 212.412 nm, 251.611 nm and 288.158 nm. Based on the ICP silicone results for each extract, the percentage of silicone deposited on the skin is calculated and is then averaged across the various time intervals at which samples are collected.

### Examples 121-140

The F series is designed as representative of a personal cleansing formulation and has the total composition reflected in Table 7.

**Table 7 - Cleanser (F Series)**

| Ingredients | % weight |
|---|---|
| **Phase A** | |
| Ammonium Lauryl Sulfate | 11.0 |
| Cocamidopropyl Betaine | 12.0 |
| Sodium C12-15 Pareth-15 Sulfate | 11.0 |

| **Phase B** | |
|---|---|
| Sodium Cocoyl Isothionate | 6.0 |
| DI Water | Balance |
| Ionic/ionogenic comb copolymer | 1.0 |
| Sodium Chloride | 3.0 |
| **Total** | 100.0 |

Each of the ionic/ionogenic comb copolymers in examples 1 to 20 is used in creating a series F formulation, the creation follows the same following procedure. First, the phase A ingredients are added together in a vessel and mixed under heating. In a separate vessel, the phase B ingredients are added together with the exception of NaCl, and are also mixed under heating. When both phases reach 45°C, the phase A materials are added to the phase B materials. The NaCl is then added and mixed until fully dissolved, at which point heat is removed and the formulation is allowed to cool to room temperature and the pH is adjusted to between 5.0 and 5.5 using triethanolamine.

### Examples 141-160

The series G formulation is designed as representative of an anti-aging formulation and has the total composition reflected in Table 8.

**Table 8 - Anti-aging cream 1 (G series)**

| Ingredients | % weight |
|---|---|
| **Phase A** | |
| DI water | Balance |
| Ionic/ionogenic comb copolymer | 1.0 |
| Niacinamide | 2.0 |

| **Phase B** | |
|---|---|
| Octinoxate | 1.0 |
| Cetearyl Alcohol | 5.5 |
| Ceteareth-25 | 0.5 |
| **Total** | 100.0 |

Each of the ionic/ionogenic comb copolymers in examples 1 to 20 is used in creating a series G formulation, the creation follows the same procedure as described for the series B formulation. Within the formulations of series G and H, the indicated amount of ionic/ionogenic comb copolymer is added on a 100% polymer basis.

### Examples 161-180

The series H formulation is designed as representative of an anti-aging formulation and has the total composition reflected in Table 9.

**Table 9 - Anti-aging cream 2 (H series)**

| Ingredients | % weight |
|---|---|
| **Phase A** | |
| DI water | Balance |
| Ionic/ionogenic comb copolymer | 1.0 |
| Erythorbic acid | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

Each of the ionic/ionogenic comb copolymers formed in examples 1 to 20 is used in creating a series H formulation, the creation follows the same procedure as described for the series B formulation.

Both the series G and H formulations are then studied to determine the effectiveness of deposition of the active anti-aging ingredient, niacinamide or erythorbic acid, using in-vitro synthetic skin. Tests are conducted by applying and rubbing 2 mg/cm² of the formulation for 30 seconds on the skin. After application, the skin is left to dry for 15 minutes, then immersed in a petri-dish with 30 mL DI water under agitation with a smooth magnetic stirrer set at a speed of 300 rpm. Water temperature is maintained at room temperature (22 °C ± 2).

At 1, 2 and 15 minutes after immersion, samples of the water are removed and filtered through a 5 micron filter. These samples are subsequently subjected to high performance liquid chromatography (HPLC) to analyze the amount of niacinamide or erythorbic acid that is extracted with the sample and thus permit calculation of the amount of anti-aging ingredientthat is deposited, which is then averaged across the various time intervals at which samples are collected.

For each of series C, and E through H, a "blank" formulation is created as a control for comparative purposes that is identical except for the exclusion of any exemplary ionic/ionogenic comb copolymer composition.

### Examples 181-200

The series I formulations are prepared by mixing 1 weight percent (on a 100% polymer basis) of each of the ionic/ionogenic comb copolymers of Examples 1 to 20 into the commercial product "head & shoulders® pyrithione zinc dandruff shampoo, classic clean" availablefrom Procter & Gamble. Mixing of the ionic/ionogenic comb copolymer and shampoo is conducted at room temperature. The commercial shampoo as purchased, without the ionic/ionogenic comb copolymer, serves as the "blank" formulation for the series I formulation.

### Examples 201-220

The series J formulations are prepared by mixing 1 weight percent (on a 100% polymer basis) of each of the ionic/ionogenic comb copolymers of Examples 1 to 20 into the commercial product "pyrithione zinc dandruff shampoo, classic clean" available from Rite Aid Corporation. Mixing of the ionic/ionogenic comb copolymer and shampoo is conducted at room temperature. The commercial shampoo as purchased, without the ionic/ionogenic comb copolymer, serves as the "blank" formulation for the series J formulation.

Both the series I and J formulations are then studied to determine the effectiveness of deposition of the active anti-dandruff ingredient, zinc pyrithione, using in-vitro synthetic skin. Tests are conducted by applying and rubbing 2 mg/cm² of the formulation for 30 seconds on the skin. After application, the skin is left to dry for 5 minutes, then immersed in a petri-dish with 30 mL DI water under agitation with a smooth magnetic stirrer set at a speed of 300 rpm. Water temperature is maintained at room temperature (22 °C ± 2).

At 30 seconds, 1 and 2 minutes after immersion, samples of the water are removed and filtered through a 5 micron filter. These samples are subsequently subjected to inductively coupled plasma atomic emission spectroscopy (ICP-OES) using a Perkin-Elmer Optima 5000 dual view emission spectrometer with axial viewing with background correction to analyze the amount of zinc pyrithione that is extracted with the sample and thus permit calculation of the amount of anti-dandruff ingredient that is deposited, which is then averaged across the various time intervals at which samples are collected.

For each of the formulations of series C, and E through J, the percentage increase in deposition achieved with respect to the comparative blank formulation is calculated.

The viscosity of formulations C, and E through J, in Pascal seconds (Pa-s) is measured employing a Brookfield rotating spindle viscometer, (Brookfield Model DV-III RV) using Helipath spindle B to E at about 10 revolutions per minute (rpm), at ambient room temperature (about 20-25 °C). Both spindle and rpm are adjusted to obtain measurements at torque readings within the recommended range for the instrument. Formulations are allowed to equilibrate at room temperature overnightprior to measurement. Three readings, each from a different location within the sample container, are taken for each sample and the results averaged.

### Examples 221-240

Formulation K is designed as representative of a high ethanol content sunscreen formulation and has the total composition reflected in Table 10.

**Table 10 - High ethanol content sunscreen (K Formulation)**

| Ingredients | % weight |
|---|---|
| Ethanol | 70.0 |
| Ionic/ionogenic comb copolymer | 5.0 |
| C12-15 Alkyl Benzoate (and) Dipropylene Glycol Dibenzoate (and) PPG-15 Stearyl Ether Benzoate | 5.0 |
| Octocrylene | 5.0 |
| Ethylhexyl salicylate | 3.0 |
| Homosalate | 5.0 |
| Butyl Methoxydibenzoylmethane | 4.0 |
| Benzophenone-3 | 3.0 |
| **Total** | 100.0 |

Each of the ionic/ionogenic comb copolymers in examples 1 to 20 is used in creating a series K formulation by addition of ingredients in the order shown at room temperature (22 °C ± 2). Within the formulations K and L, the indicated amount of ionic/ionogenic comb copolymer is added on a 100% polymer basis. Following addition of each ingredient, the mixture is stirred by hand until uniform. The pH of the formulation at 5 wt% in DI water is adjusted to between 5.0 and 5.5 using triethanolamine. A stable and homogeneous sunscreen formulation is obtained.

The SPF of each of the series K formulations is subsequently measured by IMS Inc. using the "In Vitro Water Resistance Protocol".

### Examples 241-260

Formulation L is designed as representative of a sunscreen cream formulation and has the total composition reflected in Table 11.

**Table 11 - Sunscreen cream (L Formulation)**

| Ingredients | % weight |
|---|---|
| **Phase A** | |
| Mineral Oil | 2.0 |
| Emulsifying wax NF | 5.0 |
| Steareth-21 | 1.0 |
| Steareth-2 | 0.5 |
| Cetyl Alcohol | 1.0 |
| Octinoxate | 7.5 |
| Oxybenzone | 6.0 |
| Octisalate | 5.0 |

| **Phase B** | |
|---|---|
| DI water | Balance |
| Hydroxyethyl cellulose | 0.3 |
| Ionic/ionogenic comb copolymer | 1.5 |
| Propylene glycol, diazolidinyl urea, methylparaben, propylparaben | 1.0 |
| **Total** | 100.0 |

Each of the ionic/ionogenic comb copolymers in examples 1 to 20 is used in creating a series L formulation by the following procedure. First, all of the phase A ingredients are added together in a vessel and are mixed and heated to 80°C. The DI water, hydroxyethyl cellulose and ionic/ionogenic comb copolymer are added to a separate vessel, are neutralized with TEA to pH 7.0 ± 1.0 if needed (i.e., if the pH is below 6.0), and are also heated and mixed to 80°C, at which point phase A is added to phase B. The mixture is cooled to 65°C and is homogenized at 5000 rpm for 3 min. The preservative (mixture of diazolidinyl urea and parabens in propylene glycol) is added once the formulation cools below 60 °C, and mixing is maintained until it reaches room temperature.

The SPF of each of the series L formulations is subsequently measured by IMS Inc. using the "In Vitro Water Resistance Protocol".

### Examples 261-280

The series M formulation was designed as representative of a personal care skin protective face gel formulation and had the total composition reflected in Table 12.

**Table 12 - Skin protective face gel (M series)**

| Ingredients | % Weight |
|---|---|
| DI water | Balance |
| Carbomer | 0.50 |
| Triethanolamine | 0.75 |
| Ionic/ionogenic comb coplymer | 1.0 |
| Phenyl Trimethicone | 0.50 |
| Panthenol | 0.50 |
| Cyclopentasiloxane (and) Dimethicone | 1.00 |
| DMDM Hydantoin | 0.50 |
| **Total** | 100.0 |

Each of the ionic/ionogenic comb copolymers in examples 1 to 20 was used in creating a series M formulation, the creation followed the same following procedure. First, the water was added in the vessel and begin mixing. Carbomer was slowly added into the water and was allowed to fully disperse. Remaining ingredients were added in the order listed and the formulation was allowed to mix until a homogeneous formulation was formed at room temperature and the pH was adjusted to between 5.0 and 5.5 using triethanolamine.

### Examples 281-300

The series N formulation was designed as representative of an anti-acne cream, and had the total composition reflected in Table 13.

**Table 13 - Anti-acne face cream (N series)**

| Ingredients | % Weight |
|---|---|
| **Phase A** | |
| Mineral oil | 12.5 |
| PEG-30 Dipolyhydroxystearate | 1.0 |
| Steareth-2 | 2.0 |
| Steareth-21 | 1.0 |
| Dimethicone | 4.5 |
| C12-15 Alkyl Benzoate | 4.5 |

| **Phase B** | |
|---|---|
| DI water | Balance |
| Salicylic acid | 2.0 |
| Xanthan qum | 0.9 |
| Ionic/ionoqenic comb coplymer | 1.0 |
| DMDM Hydantoin | 0.5 |
| **Total** | 100.0 |

Each of the ionic/ionogenic comb copolymers in examples 1 to 20 was used in creating a series N formulation by the following procedure. First, all of the phase A ingredients were added together in a vessel and were mixed and heated to 80°C. The DI water, xanthan gum and ionic/ionogenic comb copolymer along with salicylic acid were added to a separate vessel and were also heated and mixed to 80°C, at which point phase A was added to phase B. The mixture was cooled to 65°C and was homogenized at 5000 rpm for 3 min. The preservative DMDM hydantoin was added once the formulation cooled below 60 °C, and mixing was maintained until it reached room temperature.

### Examples 301-320

The series O formulation was designed as representative of a personal care shampoo formulation, and had the total composition reflected in Table 14.

**Table 14 - Shampoo (O series)**

| Ingredients | % Weight |
|---|---|
| DI water | Balance |
| Ionic/ionogenic comb copolymer | 2.0 |
| Triethanolamine | 0.1 |
| Cocamidopropyl betaine | 5.3 |
| Ammonium lauryl sulfate | 10.9 |
| DMDM Hydantoin | 0.5 |
| **Total** | 100.0 |

Each of the ionic/ionogenic comb copolymers in examples 1 to 20 was used in creating a series O formulation, the creation followed the same following procedure. First, the DI water was added in a vessel. While mixing, the other ingredients were added in order until a homogenous formulation was formed, after which the pH was adjusted to between 5.0 and 5.5 using triethanolamine.

### Examples 321-340

The series P formulation was designed as representative of a personal care exfoliating body wash, and had the total composition reflected in Table 15.

**Table 15 - Exfoliating body wash (P series)**

| Ingredients | % Weight |
|---|---|
| DI water | Balance |
| Ionic/ionogenic comb coplymer | 2.0 |
| Ammonium lauryl sulfate | 11.00 |
| Salicylic acid | 1.00 |
| Cocamidopropyl betaine | 5.25 |
| Glydant plus liquid | 0.25 |
| Dimethiconol (and) TEA-Dodecylbenzenesulfonate | 0.5 |
| **Total** | 100.0 |

Each of the ionic/ionogenic comb copolymers in examples 1 to 20 was used in creating a series P formulation, the creation followed the same following procedure. First, the DI water was added in a vessel. While mixing, the other ingredients were added in order until a homogenous formulation was formed, after which the pH was adjusted to between 5.0 and 5.5 using triethanolamine.

### Examples 341-360

The series Q formulation was designed as representative of a skin clarifying face cream. This formulation had the total composition reflected in Table 16.

**Table 16 - Skin clarifying face cream (Q series)**

| Ingredients | % Weight |
|---|---|
| **Phase A** | |
| Mineral oil | 12.5 |
| PEG-30 Dipolyhydroxystearate | 1.0 |
| Steareth-2 | 2.0 |
| Steareth-21 | 1.0 |
| Dimethicone | 4.5 |
| C12-15 alkyl benzoate | 4.5 |

| **Phase B** | |
|---|---|
| Water | Balance |
| Glycolic acid | 10.0 |
| Xanthan gum | 0.9 |
| Ionic/ionogenic comb coplymer | 1.0 |
| DMDM Hydantoin | 0.5 |
| **Total** | 100.0 |

Each of the ionic/ionogenic comb copolymers in examples 1 to 20 was used in creating a series Q formulation by the following procedure. First, all of the phase A ingredients were added together in a vessel and were mixed and heated to 80°C. The Dl water, xanthan gum and ionic/ionogeniccomb copolymer along with glycolic acid were added to a separate vessel and were also heated and mixed to 80°C, at which point phase A was added to phase B. The mixture was cooled to 65°C and was homogenized at 5000 rpm for 3 min. The preservative DMDM Hydantoin was added once the formulation cooled below 60 °C, and mixing was maintained until it reached room temperature.

For each of series A, B, D, and M through Q, a "blank" formulation was created as a control for comparative purposes that was identical except for the exclusion of any exemplary ionic/ionogenic comb copolymer composition.

For each of the formulations of series A and D, the percentage increase in deposition achieved with respect to the comparative blank formulation was calculated as the difference between the exemplary formulaton and the comparative blank formulation expressed as a percentage of the comparative blank formulation, with percentage increase in deposition for each of the formulations of series A and D reported in Table 17.

The viscosity of formulations A (Examples 21-40), B (Examples 41-60), D (Examples 81-100), M (Examples 261-280), N (Examples 281-300), O (Examples 301-320), P (Examples 321-340) and Q (Examples 341-360), in Pascal seconds (Pa-s) was measured employing a Brookfield rotating spindle viscometer, (Brookfield Model DV-III RV) using Helipath spindle B to E at about 10 revolutions per minute (rpm), at ambient room temperature (about 20-25 °C). Both spindle and rpm were adjusted to obtain measurements at torque readings within the recommended range for the instrument. Formulations were allowed to equilibrate at room temperature overnight prior to measurement. Three readings, each from a different location within the sample container, were taken for each sample and the results averaged and reported in Table 17. Where vacancies appear in Table 17, no measurement was obtained.

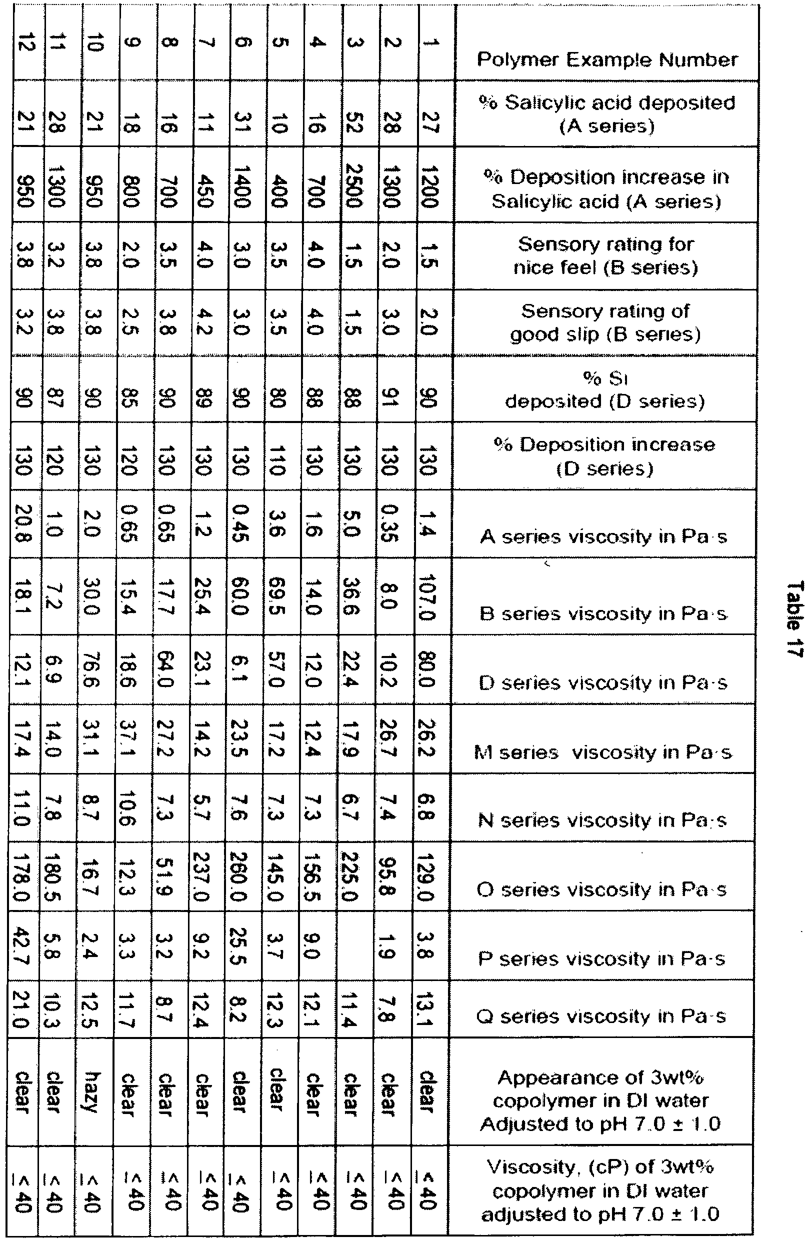

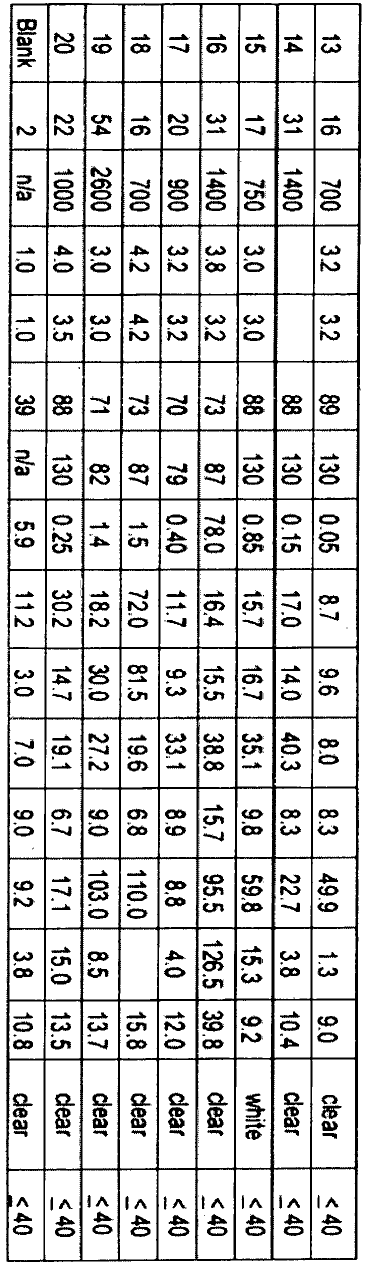

### Brief Discussion of Results of Experimental Personal Care Formulations

The results compiled in Table 17 demonstrated a significant increase in deposition of the active ingredients (salicylic acid and silicone) for every ionic/ionogenic comb copolymer in both the series A and series D formulations. Likewise, the sensory ratings for feel and slip were in each case at least equivalent to and typically superior in comparison to the "blank" formulation.

Increased thickening of the formulations compared to the blank formulations was achieved in many cases. Because the formulations exhibited significant enhancement of active deposition and generally superior sensory effects, in cases where thickening was not observed, a thickening agentcould be used as an additive in those cases. Suitable additive thickening agents, include for example but are not limited to, cellulosic thickeners including untreated cellulose, chemically modified cellulosics (e.g.: cellulose ethers, hydroxyethyl cellulose (HEC), methylcellulose, carboxymethyl cellulose), natural gums (e.g.: xanthan gum, guar gum), starches and modified starches (e.g.: aluminum starch octenylsuccinate), clays, hydrophobically modified acrylates and acrylate thickeners (e.g.: carbomer), and associative thickeners; such optional thickening agents typically added to a personal care formulation at about 0.1wt% to about 5wt%. While additives can be used to achieve thickening, deposition and sensory attributes cannot readily be achieved by the inclusion of additives, illustrating the effectiveness of ionic/ionogenic comb copolymers in accordance with exemplary embodiments which can achieve such results. In certain examples, increased thickening of low pH personal care formulations containing example ionic/ionogenic oomb copolymers was achieved as compared to the blank formulations.

In fact, for situations in which the copolymer did exhibit thickening of the overall formulation, in each case where the viscosity was measured of the copolymer sample in DI water alone (at 3 wt% solids copolymer, neutralized to pH 7.0 ± 1.0 with TEA), the viscosity was less than the viscometer's lowest measurement capability (40 cP), which demonstrates non-thickening of water. While not wishing to be bound by theory or explanation, it is believed that the ionic/ionogenic comb copolymers in accordance with exemplary embodiments collapse in water, but not within the personal care product formulations as a result of interactions due to the presence of other organic constituents.

### Discussion of Other Products in which Copolymer May be Employed

Although described primarily in conjunction for use with personal care products, it will be appreciated that ionic/ionogenic comb copolymer compositions in accordance with exemplary embodiments may also be useful as an ingredient for other commercial applications.

Exemplary applications include cleaning products, used in household, industrial and/or institutional settings such as surface cleansers used for cleaning kitchens and bathrooms (e.g. cleaning countertops and tiled surfaces), toilet cleansers, toilet bowl rim cleansers, floor cleansers, wall cleansers, vehicle cleansers, air fresheners, dishwasher detergents and other dish soaps, laundry treatments and detergents, fabric softeners, spot reducers, fabric treatments and the like, all by way of example only. Other uses may include the use of such copolymers as ingredients in metal cleaners, scale removers, paint and varnish strippers, and polishes for furniture, shoes, cars, or metals, again all by way of example only.

The ionic/ionogeniccomb copolymers disclosed herein are also suitable for use in various industrial processes and applications. Exemplary applications include the use of such polymers as processing and finishing aids for textile dyeing/coating; in printing and finishing formulation inks; as stabilizers for chemical processes (e.g. when polymerizations are carried out in aqueous solution or emulsion, such as the preparation of photographic emulsions); as flocculants for wastewater treatment, as auxiliaries for papermaking such as the production of papers for use with inkjet printers, and as a humectant or gel former, for example.

As with the personal care products, it will further be appreciated that these other types of products including the ionic/ionogeniccomb copolymers disclosed herein can be in any form, including but not limited to, liquid, gel, spray, emulsion, semisolid (such as pastes), and solid (such as stick, tablet or bar form), as may be desirable for the intended function of the particular composition.

The invention has been described with reference to certain aspects, but other aspects and embodiments are apparent to persons of skill in the art, and are included within the scope of the claims.

## Claims

1. A comb copolymer comprising:
A) one or more repeating units derived from at least one member selected from the group consisting of olefinically unsaturated anionic, anionogenic and zwitterionic comonomers; wherein anionic monomers includes salts of at least one of monomers with carboxylic or dicarboxylic acid groups, anhydrides, salts of phosphoric acid and salts of half-esters of dicarboxylic acids and
B) one or more repeating units having the formula 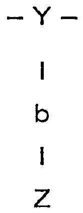 wherein Y is a moiety forming part of the copolymer backbone, Z is a moiety that exhibits association with other Z moieties or with other moieties within a formulation in which the copolymer will be employed, and b is a bond or moiety that links the Z moiety to the Y moiety; wherein the concentration of ionic and ionogenic repeats in the copolymer is between 10 and 400 meq per 100 grams of polymer, the weight of associative moieties Z is between 3 and 30 grams per 100 grams of polymer and the ratio of associative moieties Z to ionic and ionogenic repeats is between 0.025 and 1.3

2. The comb copolymer of Claim 1, further comprising one or more repeating units derived from a monomer selected from at least one of the following groups:
C) acrylamide monomers;
D) one or more olefinically unsaturated hydrophilic monomers that are not A, B or C; or
E) one or more olefinically unsaturated monomers that are not A, B, C or D.

3. The comb copolymer of Claim 2, comprising one or more repeating units derived from monomers selected from at least two of groups C, D or E.

4. The comb copolymer of Claim 3, comprising one or more repeating units derived from monomers selected from each of groups C, D and E.

5. The comb copolymer of Claim 1, wherein the repeating units of Group B comprise surfactant repeating units.

6. The comb copolymer of Claim 1, wherein Z comprises at least one member selected from the group consisting of alkyl, fluoroalkyl, silicone and silane.

7. The comb copolymer of Claim 1, wherein A comprises at least one comonomer having a formula: 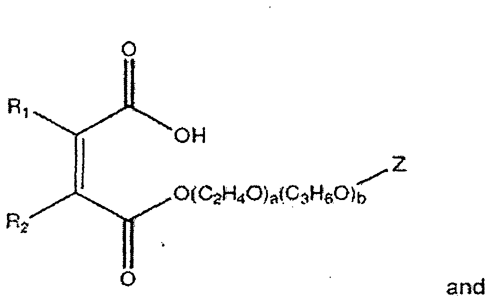 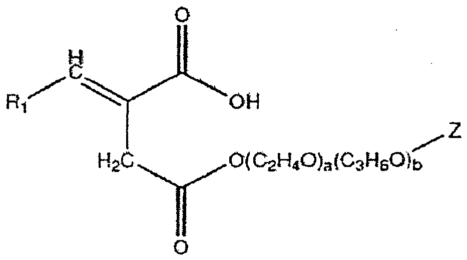 R₁ and R₂ are independently either H or CH₃; a is between 0-50 and b is between 0-50.

8. The comb copolymer of Claim 1 wherein a three percent (solids) weight solution of the comb copolymer in water at pH 7.0 ± 1.0 has a viscosity at 25 °C of less than 250 cP.

9. A personal care product comprising
a cosmetic base media; and
0.1% to 20% by weight of the comb copolymer of any one of claims 1 to 4, or of any one of claims 1 to 8.

10. The personal care product of claim 9, further comprising an active ingredient, wherein the comb copolymer increases the deposition of the active ingredient to a keratinous substrate by at least 10% over a personal care product having a same formulation without the comb copolymer.

11. A personal care composition comprising:
a cosmetic base media and at least one comb copolymer of any one of claims 1 to 8.

12. The composition of Claim 11 comprising at least two repeating units.

13. The composition of Claim 11, wherein A comprises zwitterionic comonomers.

14. The composition of Claim 11, wherein the repeating units of Group B comprise surfactant repeating units.

15. The composition of Claim 11, wherein Z comprises at least one member selected from the group consisting of alkyl, fluoroalkyl, silicone and silane.

16. The composition of Claim 11, wherein comonomer A comprises at least one comonomer having a formula: 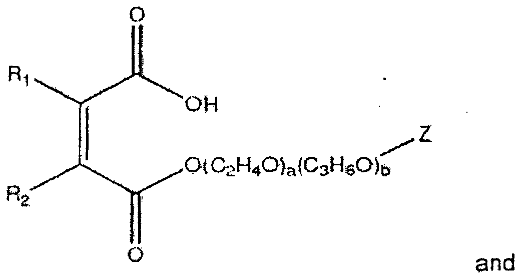 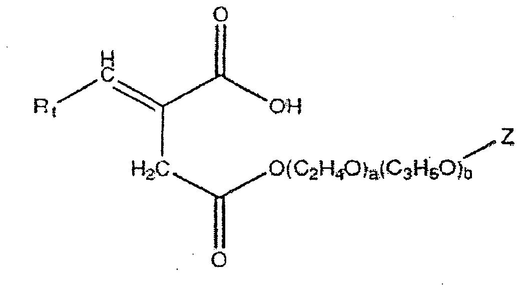 R₁ and R₂ are independently either H or CH₃; a is between 0-50 and b is between 0-50.

17. The composition of Claim 11, wherein Z comprises 10 to 18 carbon atoms.

18. The composition of Claim 11, wherein the repeating units of Group B comprise hydrophobic repeating units.

19. The composition of Claim 11, wherein b comprises at least one of urethane and urea linkages.

20. The composition of Claim 11, wherein Group B comprises at least one of sulphonic and phosphonic acid repeating units.

21. The composition of Claim 11, wherein the comb copolymer is not crosslinked.

## Patentansprüche

1. Kammcopolymer, umfassend:
A) ein oder mehrere Wiederholungseinheiten, die sich von mindestens einem Mitglied ableiten, das aus der Gruppe ausgewählt ist, bestehend aus olefinisch ungesättigten anionischen, anionogenen und zwitterionischen Comonomeren, wobei die anionischen Monomere Salze von mindestens einem der Monomere mit Carbonsäure- oder Dicarbonsäuregruppen, Anhydride, Salze der Phosphorsäure und Salze von Halbestern der Dicarbonsäuren einschließen, und
B) ein oder mehrere Wiederholungseinheiten, die die Formel 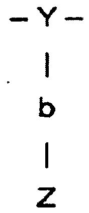 haben,
wobei Y eine Hälfte ist, die einen Teil des Copolymer-Rückgrats bildet, Z eine Hälfte ist, die eine Assoziation mit anderen Z-Hälften oder mit anderen Hälften innerhalb einer Formulierung aufweist, in der das Copolymer eingesetzt werden wird, und b eine Bindung oder Hälfte ist, die die Z-Hälfte an die Y-Hälfte bindet; wobei die Konzentration der ionischen und ionogenen Wiederholungen in dem Copolymer zwischen 10 und 400 meq pro 100 Gramm Polymer ist, wobei das Gewicht der assoziativen Hälften Z zwischen 3 und 30 Gramm pro 100 Gramm Polymer ist und wobei das Verhältnis der assoziativen Hälften Z zu ionischen und ionogenen Wiederholungen zwischen 0,025 und 1,3 ist.

2. Kammcopolymer nach Anspruch 1, ferner umfassend ein oder mehrere Wiederholungseinheiten, die sich von einem Monomer ableiten, das aus mindestens einer der folgenden Gruppen ausgewählt ist:
C) Acrylamidmonomere;
D) ein oder mehrere olefinisch ungesättigte hydrophile Monomere, die nicht A, B oder C sind; oder
E) ein oder mehrere olefinisch ungesättigte Monomere, die nicht A, B, C oder D sind.

3. Kammcopolymer nach Anspruch 2, umfassend ein oder mehrere Wiederholungseinheiten, die sich von Monomeren ableiten, die zumindest aus zwei der Gruppen C, D oder E ausgewählt sind.

4. Kammcopolymer nach Anspruch 3, umfassend ein oder mehrere Wiederholungseinheiten, die sich von Monomeren ableiten, die aus jeder der Gruppen C, D und E ausgewählt sind.

5. Kammcopolymer nach Anspruch 1, wobei die Wiederholungseinheiten der Gruppe B Wiederholungseinheiten eines oberflächenaktiven Stoffs umfassen.

6. Kammcopolymer nach Anspruch 1, wobei Z mindestens ein Mitglied ausgewählt aus der Gruppe, bestehend aus Alkyl, Fluoralkyl, Silikon und Silan, umfasst.

7. Kammcopolymer nach Anspruch 1, wobei A mindestens ein Comonomer umfasst, das eine Formel 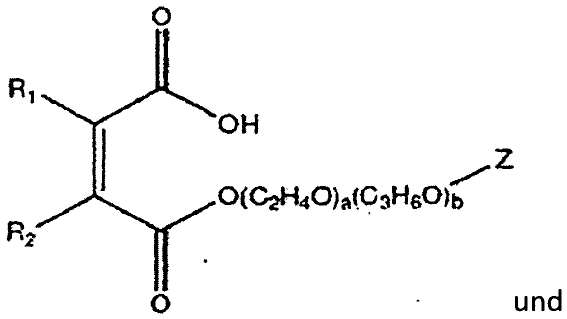 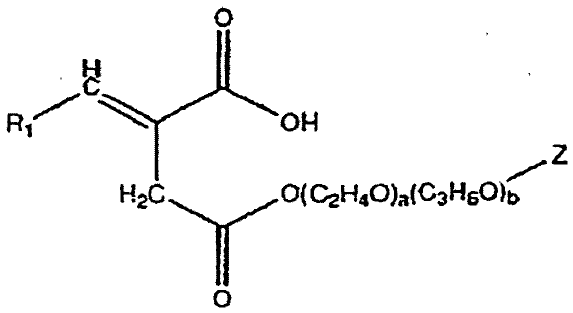 hat,
wobei R₁ und R₂ unabhängig entweder H oder CH₃ sind; a zwischen 0-50 ist und b zwischen 0-50 ist.

8. Kammcopolymer nach Anspruch 1, wobei eine drei-gewichtsprozentige (Feststoffe) Lösung des Kammcopolymers in Wasser bei pH 7,0 ± 1,0 und 25 °C eine Viskosität von weniger als 250 cP hat.

9. Körperpflegeprodukt, umfassend
ein kosmetisches Basismedium; und
0,1 Gew.-% bis 20 Gew.-% des Kammcopolymers nach einem der Ansprüche 1 bis 4 oder nach einem der Ansprüche 1 bis 8.

10. Körperpflegeprodukt nach Anspruch 9, ferner umfassend einen aktiven Bestandteil, wobei das Kammcopolymer die Deposition des aktiven Bestandteils auf ein keratinöses Substrat um mindestens 10 % gegenüber einem Körperpflegeprodukt erhöht, das eine gleiche Formulierung aber ohne das Kammcopolymer hat.

11. Körperpflegezusammensetzung, umfassend:
ein kosmetisches Basismedium und mindestens ein Kammcopolymer nach einem der Ansprüche 1 bis 8.

12. Zusammensetzung nach Anspruch 11, umfassend mindestens zwei Wiederholungseinheiten.

13. Zusammensetzung nach Anspruch 11, wobei A zwitterionische Comonomere umfasst.

14. Zusammensetzung nach Anspruch 11, wobei die Wiederholungseinheiten der Gruppe B Wiederholungseinheiten eines oberflächenaktiven Stoffs umfassen.

15. Zusammensetzung nach Anspruch 11, wobei Z mindestens ein Mitglied ausgewählt aus der Gruppe, bestehend aus Alkyl, Fluoralkyl, Silikon und Silan, umfasst.

16. Zusammensetzung nach Anspruch 11, wobei das Comonomer A mindestens ein Comonomer umfasst, das eine Formel 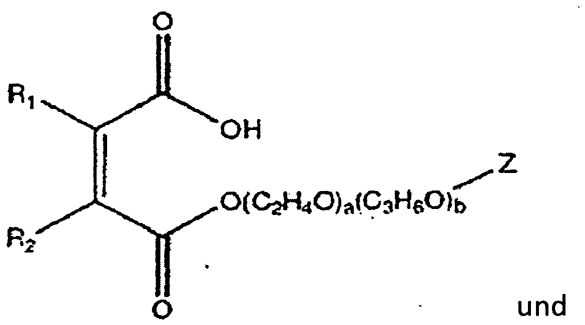 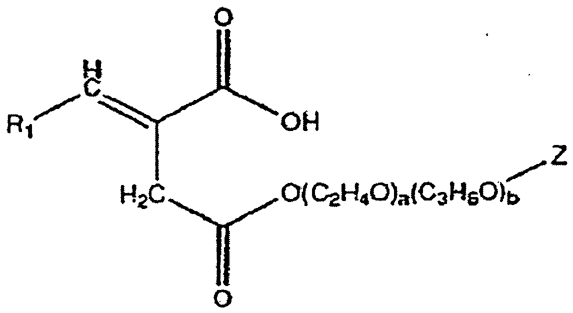 hat,
wobei R₁ und R₂ unabhängig entweder H oder CH₃ sind; a zwischen 0-50 ist und b zwischen 0-50 ist.

17. Zusammensetzung nach Anspruch 11, wobei Z 10 bis 18 Kohlenstoffatome umfasst.

18. Zusammensetzung nach Anspruch 11, wobei die Wiederholungseinheiten der Gruppe B hydrophobe Wiederholungseinheiten umfassen.

19. Zusammensetzung nach Anspruch 11, wobei b mindestens eine aus Urethan- und Harnstoffverbindungen umfasst.

20. Zusammensetzung nach Anspruch 11, wobei die Gruppe B mindestens eine aus Sulfonsäure- und Phosphonsäure-Wiederholungseinheiten umfasst.

21. Zusammensetzung nach Anspruch 11, wobei das Kammcopolymer nicht quervernetzt ist.

## Revendications

1. Copolymère en peigne comprenant :
A) un ou plusieurs motifs de répétition dérivés d'au moins un élément choisi dans le groupe constitué de comonomères oléfiniquement insaturés anioniques, anionogènes et zwitterioniques ; les monomères anioniques comprenant des sels d'au moins un des monomères avec des groupes acide carboxylique ou dicarboxylique, des anhydrides, des sels d'acide phosphorique et des sels de semi-esters d'acides dicarboxyliques et
B) un ou plusieurs motifs de répétition ayant la formule 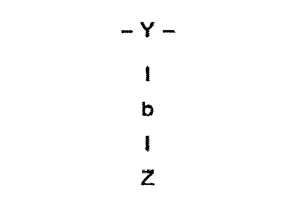 dans laquelle Y est un fragment faisant partie du squelette du copolymère, Z est un fragment qui présente une association avec d'autres fragments Z ou avec d'autres fragments dans une formulation dans laquelle le copolymère est destiné à être utilisé, et b est une liaison ou un fragment qui lie le fragment Z au fragment Y ; dans lequel la concentration de répétitions ioniques et ionogènes dans le copolymère est compris entre 10 et 400 meq par 100 grammes de polymère, le poids de fragments associatifs Z est compris entre 3 et 30 grammes par 100 grammes de polymère et le rapport de fragments associatifs Z aux répétitions ioniques et ionogènes est compris entre 0,025 et 1,3.

2. Copolymère en peigne de la revendication 1, comprenant en outre un ou plusieurs motifs de répétition dérivés d'un monomère choisi parmi au moins un des groupes suivants :
C) des monomères d'acrylamide ;
D) un ou plusieurs monomères hydrophiles à insaturation oléfinique qui ne sont pas A, B ou C ; ou
E) un ou plusieurs monomères à insaturation oléfinique qui ne sont pas A, B, C ou D.

3. Copolymère en peigne de la revendication 2, comprenant un ou plusieurs motifs de répétition dérivés de monomères choisis parmi au moins deux des groupes C, D ou E.

4. Copolymère en peigne de la revendication 3, comprenant un ou plusieurs motifs de répétition dérivés de monomères choisis parmi chacun des groupes C, D et E.

5. Copolymère en peigne de la revendication 1, dans lequel les motifs de répétition du groupe B comprennent des motifs de répétition de tensioactif.

6. Copolymère en peigne de la revendication 1, dans lequel Z comprend au moins un élément choisi dans le groupe constitué d'alkyle, fluoroalkyle, silicone et silane.

7. Copolymère en peigne de la revendication 1, dans lequel A comprend au moins un comonomère ayant une formule : 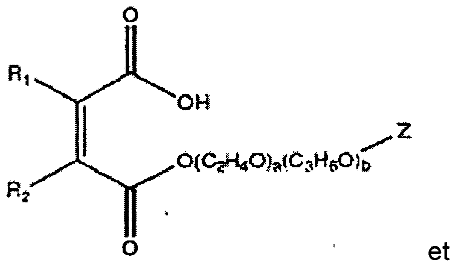 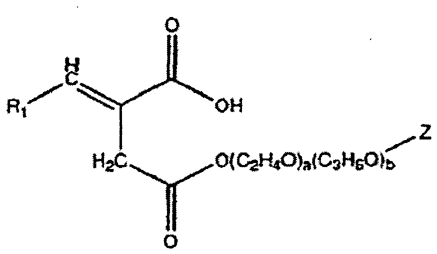 R₁ et R₂ sont indépendamment H ou CH₃ ; a est compris entre 0 et 50 et b est compris entre 0 et 50.

8. Copolymère en peigne de la revendication 1 dans lequel une solution à trois pour cent (de solides) en poids du copolymère en peigne dans l'eau à pH 7,0 ± 1,0 a une viscosité à 25 °C inférieure à 250 cP.

9. Produit de soin personnel comprenant un milieu de base cosmétique ; et 0,1% à 20 % en poids du copolymère en peigne de l'une quelconque des revendications 1 à 4, ou de l'une quelconque des revendications 1 à 8.

10. Produit de soin personnel de la revendication 9, comprenant en outre une substance active, dans lequel le copolymère en peigne augmente le dépôt de la substance active sur un substrat kératinique d'au moins 10 % par rapport à un produit de soin personnel ayant la même formulation sans le copolymère en peigne.

11. Composition de soin personnel comprenant :
un milieu de base cosmétique et au moins un copolymère en peigne de l'une quelconque des revendications 1 à 8.

12. Composition de la revendication 11 comprenant au moins deux motifs de répétition.

13. Composition de la revendication 11, dans laquelle A comprend des comonomères zwitterioniques.

14. Composition de la revendication 11, dans laquelle les motifs de répétition de groupe B comprennent des motifs de répétition de tensioactif.

15. Composition de la revendication 11, dans laquelle Z comprend au moins un élément choisi dans le groupe constitué d'alkyle, fluoroalkyle, silicone et silane.

16. Composition de la revendication 11, dans laquelle le comonomère A comprend au moins un comonomère ayant une formule : 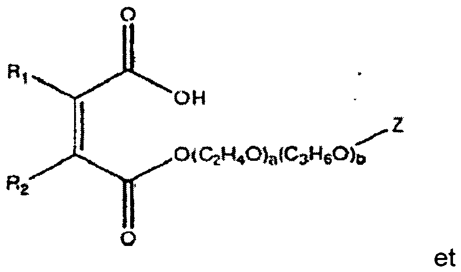 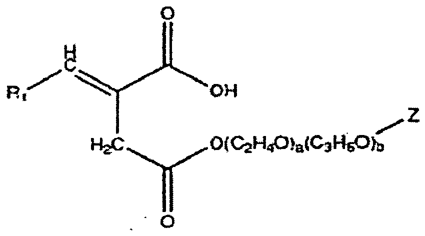 R₁ et R₂ sont indépendamment H ou CH₃ ; a est compris entre 0 et 50 et b est compris entre 0 et 50.

17. Composition de la revendication 11, dans laquelle Z comprend 10 à 18 atomes de carbone.

18. Composition de la revendication 11, dans laquelle les motifs de répétition du groupe B comprennent des motifs de répétition hydrophobes.

19. Composition de la revendication 11, dans laquelle b comprend au moins l'un de lieurs uréthane et urée.

20. Composition de la revendication 11, dans laquelle le groupe B comprend au moins l'un de motifs de répétition acide sulfonique et phosphonique.

21. Composition de la revendication 11, dans laquelle le copolymère en peigne n'est pas réticulé.
